# EUROPEAN PATENT SPECIFICATION

(11) **EP 2 531 220 B1**
(45) Date of publication and mention of the grant of the patent: **02.05.2018**
(21) Application number: 10838218.5
(22) Date of filing: 15.12.2010
(51) Int. Cl.: A61K 47/30, A61K 47/38, A61K 33/08, A61K 39/395, A61K 38/08, A61K 38/06, A61K 9/22, A61K 9/127, A61L 27/38, A61L 27/44, A61L 27/54, A61L 27/56, A61K 9/00, A61K 41/00, A61K 9/06, A61K 47/36, A61K 9/50, A61L 27/50

(54) **ACTIVE SCAFFOLDS FOR ON-DEMAND DRUG AND CELL DELIVERY**
AKTIVE GERÜSTE FÜR ON-DEMAND-FREISETZUNG VON WIRKSTOFFEN UND ZELLEN
ÉCHAFAUDAGES ACTIFS POUR ADMINISTRATION DE MÉDICAMENT ET DE CELLULE À LA DEMANDE

(30) Priority: 18.12.2009 US 288028 P
(43) Date of publication of application: 12.12.2012
(73) Proprietor: President and Fellows of Harvard College, Cambridge, MA 02138 (US)
(72) Inventor: MOONEY, David J., Sudbury, Massachusetts 01776 (US); ZHAO, Xuanhe, Cambridge, Massachusetts 02138 (US)
(74) Representative: Kehoe, Laura Ellen
(86) International application number: PCT/US2010/060456
(87) International publication number: WO 2011/075516

(56) References cited:
- WO-A1-2007/065933
- WO-A1-2011/086210
- WO-A2-01/72281
- WO-A2-2006/116752
- US-A- 5 714 536
- US-A1- 2009 053 512

## Description

### GOVERNMENT SUPPORT

This invention was made with government support under grant no. DMR-0820484 awarded by the National Science Foundation and under grant no. DE013033 and no. R01 DE019917 awarded by the National Institutes of Health. The government has certain rights in this invention.

### FIELD OF THE INVENTION

The invention relates to materials that can vary their porosity, pore size, pore connectivity, swelling agent concentration, and/or specific volume in response to an external stimuli. This invention further relates to use of these materials for control release drug and cell delivery.

### BACKGROUND OF THE INVENTION

For over two decades, porous biomaterials fabricated from natural and synthetic polymers, metals, ceramics and glasses have been intensively studied and widely used as drug delivery vehicles and scaffolds for tissue regeneration (Langer, R. & Vacanti, J. P. Science, 1993, 260, 920-926; Griffith, L. G. & Naughton, G. Science, 2002, 295, 1009-1016; Hutmacher, D. W. Biomaterials, 2002, 21, 2529-2543; Karageorgiou, V. & Kaplan, D. Biomaterials, 2005, 26, 5474-5491; and Hollister, S. J. Nat. Mater. 2005, 4, 518-524). For example, collagen sponges that release bone morphogenetic proteins are widely used in spine surgery, with an approximately billion-dollar market (Mroz, T., Yamashita, T., & Lieberman, I. Spine J. 2008, 8, 41S-42S). The porous scaffolds provide a three-dimensional environment that preserves tissue volume, supports cell interactions, and delivers biological agents (Mikos, et al., Polymer, 1994, 35, 1068-1077; Mooney, et al., Biomaterials, 1996, 17, 1417-1422; Shastri, V. P., Martin, I., & Langer, R., Proc. Natl. Acad. Sci. U. S. A. 2000, 97, 1970-1975; and Yang, et al., Tissue Eng. 2001, 7, 679-689). More recently, porous scaffolds have shown great promise as carriers in cell therapy in the treatment of a variety of diseases (Hofmann, et al., Circulation, 2005, 111, 2198-2202; Skuk, et al., J. Neuropathol. Exp. Neurol. 2003, 62, 951-967; Evans, S. M., Mummery, C., & Doevendans, P. A., Semin. Cell Dev. Biol. 2007, 18, 153-160; and Thuret, S., Moon, L. D. F., & Gage, F. H., Nat. Rev. Neurosci. 2006, 7, 628-643). Significant improvement in cell viability, engraftment, and control over cell fate is possible by delivering cells with the porous scaffolds, in contrast to cell injections or infusions (Auger, et al., Biotechnol. Appl. Biochem. 2004, 39, 263-275; Nerem, R. M. Biomaterials, 2007, 28, 5074-5077 and Mooney, D. J. & Vandenburgh, H., Cell Stem Cell, 2008, 2, 205-213). In all these cases, it is highly desirable to trigger and/or regulate the delivery of biological agents (e.g. drugs and cells) with external cues, as dynamical control over delivery can potentially improve the safety and efficiency of the agents, and permit new therapies (Langer, R. Science, 1990, 249, 1527-1533).

In the field of drug delivery, active biomaterials that are responsive to external stimuli such as temperature, pH, enzymes and various physical fields have been extensively explored for controlled delivery (Hoffman, A. S. Artificial Organs, 1995, 19, 458-467; Hsieh, D. S. T., Langer, R., & Folkman, J., Proc. Nat. Acad. Sci. U.S.A. 1981, 78, 1863-1867; Peppas, et al., Adv. Mater. 2006, 18, 1345-1360; Galaev, I. Y. & Mattiasson, B., Trends Biotechnol. 1999, 17, 335-340; Miyata, T., Asami, N., & Uragami, T., Nature, 1999, 399, 766-769; and Wood, et al., Proc. Natl. Acad. Sci. U. S. A. 2008, 105, 2280-2285. On the other hand, the porous scaffolds currently used in tissue engineering and cell therapy are mostly passive in that they deliver biological agents mainly through mechanisms involving molecular diffusion, material degradation, and cell migration, which do not allow for dynamic external regulations. WO 2006/116752 discloses compositions comprising nano structures for cell, tissue and artificial organ growth. WO 2007/065933 discloses magnetic polymer particles.

Stimuli-responsive polymers represent one class of actuators that have the unique ability to change swelling behaviors, permeability, and elasticity in a reversible manner. Owing to these useful properties, stimuli-responsive polymers have numerous applications, particularly in medicine, pharmaceutics, drug-delivery, biosensors, separation and membranes, purifications, and enzyme and cell immobilization (Qiu, Y. and Park, K., Adv Drug Delivery Rev. 2001, 53, 321-339; Miyata, T. Urgami, T. and Nakamae, K., Adv. Drug Delivery Rev. 2002, 54, 79-98; Bckiari, V. et al., Langmuir, 2004, 20, 7972; Lopes, D. Cendoya, L. and Mijangos, C., Macromol .Symp. 2001, 166, 173; Lao, L. and Ramanujan, V.R., J. Mater. Sci. Mater. Med. 2004, 15, 1061; Porter, J. and Pickup, R.W.J., Microbiol. Methods, 1998, 33, 221; Xie, X. et al., J. Magn. Mater. 2004, 227, 16; and Gupta, P.K. and Hung, C.T., Life Sci. 1989, 44, 175.

Magnetic gels, also called ferrogels, are a class of stimuli-responsive polymeric materials with their properties controlled by magnetic field. Ferrogels contain magnetic particles dispersed homogenously or heterogeneously and confined in a polymer network. Under a non-uniform magnetic field, particles undergo motion, which in turn induces elongation, contraction, or bending of the gels with short response time (Torok, G. et al., Phys. B, 2001, 297, 40; Szabo, D. Czako-Nagu, I. and Zrinyi, M., J Colloid Interface Sci. 2002, 221, 166; Szabo, D. Szeghy, G and Zrinyi, M., Macromolecules, 1998, 31, 6541; Xulu, P. Filipesei, G. and Zrinyi, M., Macromolecules, 2000, 33, 1716; Zrinyi, M. Feher, J. and Filipesei, G., Macromolecules, 2000, 33, 5751; Zrinyi, M. Barsi, L. and Buki, A., J. Chem. Phys. 1996, 104, 8750; and Zrinyi, M. Barsi, L. and Buki, A., Polym. Gels Networks, 1997, 5, 415). Although magnetic gels under go changes with short response time, these changes, especially changes in specific volume, are not substantial.

Accordingly, it would be desirable to have materials that can undergo substantial specific volume changes in response to an external stimuli.

### SUMMARY OF THE INVENTION

In one aspect, the invention provides a composition comprising a matrix material and a magnetic material distributed in the matrix material, wherein the matrix material comprises pores having mean pore diameter in range of about 100 µm to about 1000µm, and wherein porosity, pore size, pore connectivity, and/or specific volume of the matrix material undergoes a change from a first value to a second value in response to an electromagnetic signal.

Also, described herein, is a method for releasing a therapeutic agent or a cell on demand in response to an external stimuli, the method comprising: (a) providing to a subject a composition described herein, wherein the composition contains the therapeutic agent or the cell; and (b) inducing a change in porosity, pore size, pore connectivity, swelling agent concentration, and/or specific volume of the composition via a magnetic field to release the therapeutic agent or the cell.

### BRIEF DESCRIPTION OF THE DRAWINGS

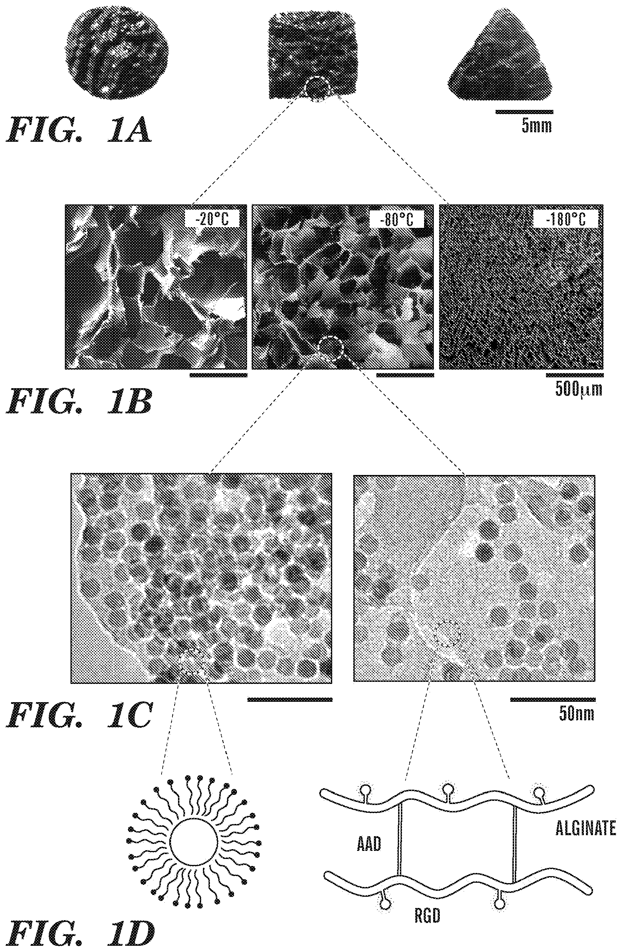

**Figs. 1A-1D** show the hierarchical structure of macroporousferrogels. **Fig. 1A** are photographs of bulk gels with various shapes. **Fig. 1B** shows the SEM images of scaffold with various size pores (average diameter from left to right: 700µm, 300µm and 20µm) and pore connectivity (from left to right: good, good, poor) in the ferrogels. **Fig. 1C** shows the TEM images of iron oxide nanoparticles in the gel at predetermined concentrations (left 13 wt %, and right 4 wt %). **Fig. 1D** shows the schematic plots of the nanoparticles coated with Pluronic F127 (left), and alginate covalently crosslinked by AAD and coupled with RGD peptides (right). Size bars are shown on images.

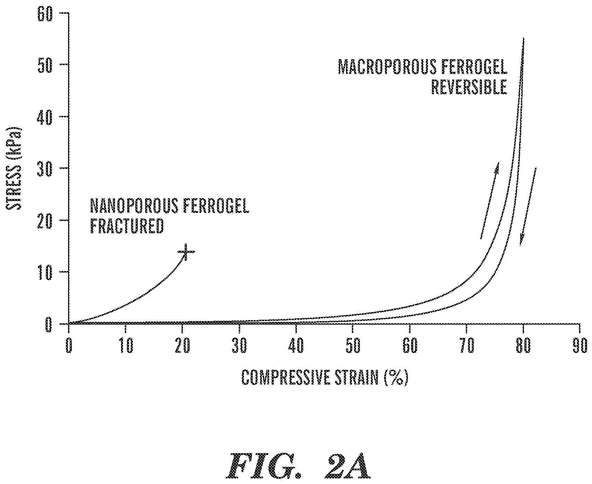

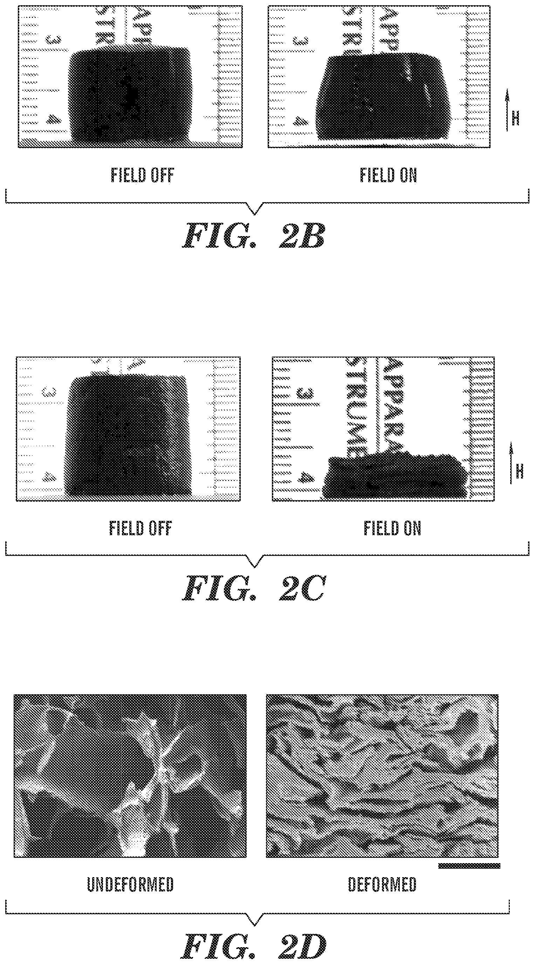

**Figs. 2A-2D** show the characteristics of the magnetic gels. **Fig. 2A** is a line graph showing stress vs. strain curves for nanoporousferrogel and macroporousferrogel subjected to compression tests. **Figs. 2B** and **2C** are photographs showing the effect of a magnetic field on a cylinder of a nanoporousferrogel (**Fig. 2A**) and the corresponding macroporousferrogel (**Fig. 2C**). The nanoporous gel reduced its height ∼5% when subjected to a vertical magnetic-field gradient of ∼38 A/m²; the corresponding macroporousferrogel deformed ∼70% under the same magnetic field. **Fig. 2D** shows the SEM images of a freeze-dried macroporousferrogel in the undeformed and deformed states. The size bar is 500µm.

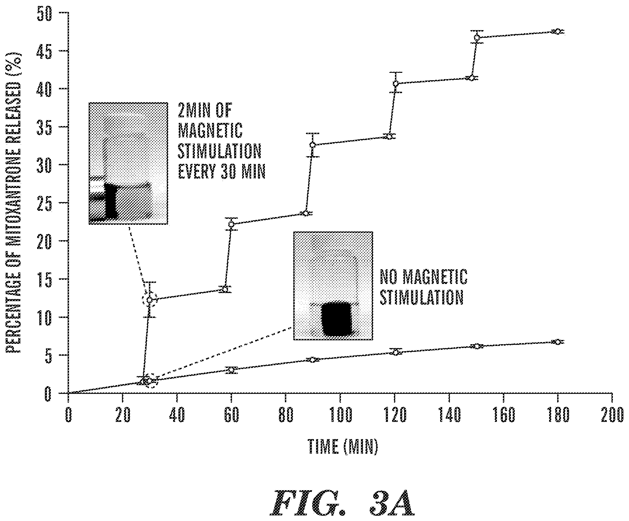

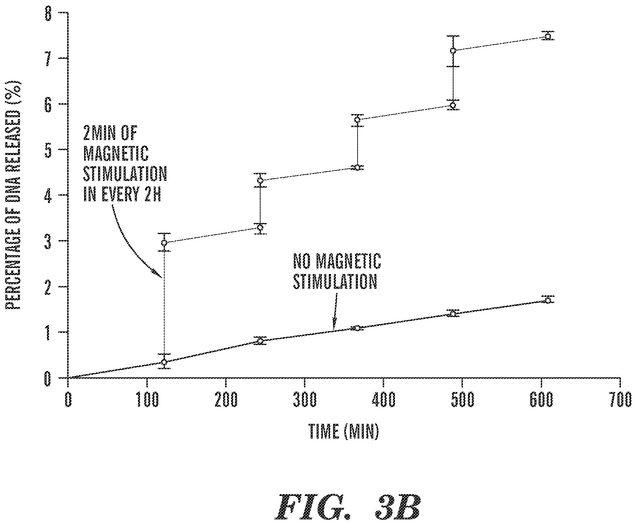

**Figs 3A -3B** are line graph showing the cumulative release profiles of mitoxantrone (**Fig. 3A**) and plasmid DNA (**Fig. 3B**) from macroporousferrogels. In **Fig. 3A****,** the macroporous gel was subjected to 2 min of magnetic stimulation every 30 min for, or no magnetic stimulation. In **Figs. 3B** the macroporous gel was subjected to 2 min of magnetic stimulation every 2 hours, or no magnetic stimulation. Values represent mean and standard deviation of each increment (n=3-5).

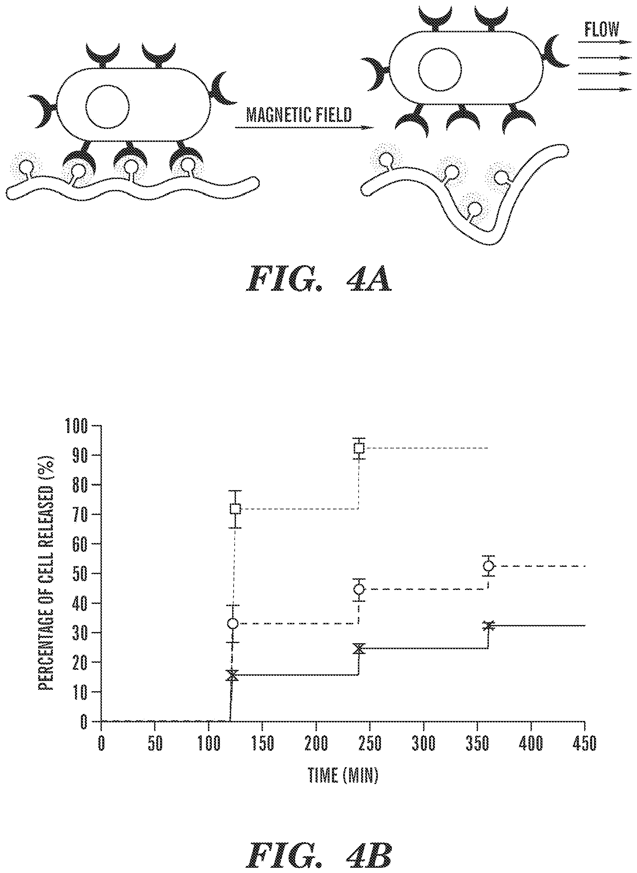

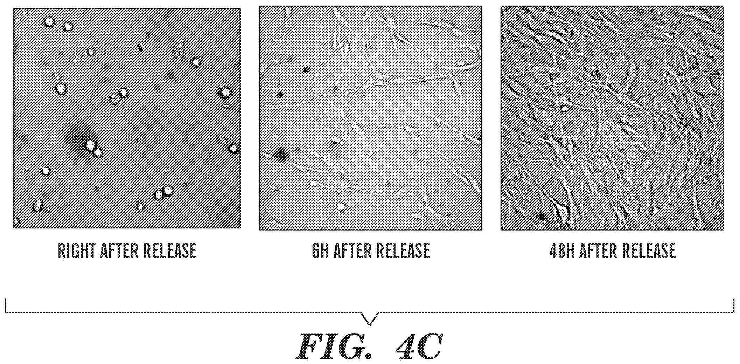

**Figs. 4A-4C** show the release of cells from macroporous gels. **Fig. 4A** is a schematic plot of gel deformation and resulting water convection inducing cell release from macroporous gels (Dainiak, et al., Proc. Natl. Acad. Sci. U. S. A. 2006, 103, 849-854). **Fig. 4B** is a line graph showing the cumulative release profiles of fibroblasts from macroporousferrogels with 100% (-), 50% (- - - -), and 10% (- · - · - · -) of the baseline RGD density, following application of cycled magnetic field. Values represent mean and standard deviation of each increment (n=3-5). Differences between the values of cell release at the various RGD densities were statistically significant at each time point (p<0.05). **Fig. 4C** is photographs showing the spreading and proliferation of the released cells at various times after replating onto tissue culture plastic.

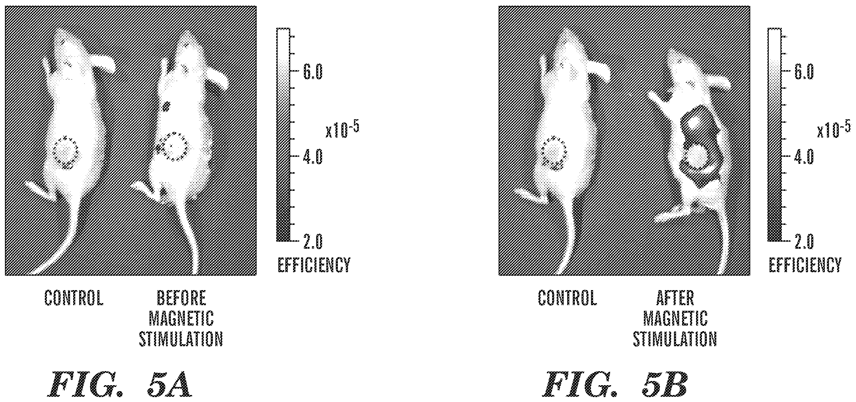

**Fig. 5A and 5B** are photographs showing *in-vivo* fluorescence images of mice implanted with macroporousferrogels containing mouse mesenchymal stem cells stained with DiOC18 before (**Fig. 5A**) and after (**Fig. 5B**) magnetic stimulation. The control case was subject to no magnetic stimulation. The positions of the gel disks are indicated by circles on the figure.

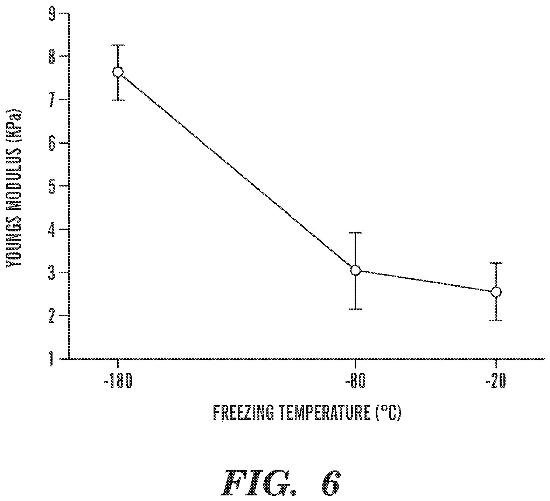

**Fig. 6** is a line graph showing the initial Young's moduli of macroporousferrogels prepared at various freezing temperatures. Values represent mean and standard deviation (n=3).

### DETAILED DESCRIPTION OF THE INVENTION

In one aspect, the invention provides a composition comprising a matrix material and a magnetic material distributed in the matrix material, wherein the matrix material comprises pores having mean pore diameter in range of about 10 µm to about 1000µm, and wherein porosity, pore size, pore connectivity, and/or specific volume of the matrix material undergoes a change from a first value to a second value in response to an electromagnetic signal. It is to be understood that, change in one of porosity, pore size, pore connectivity, swelling agent concentration, and/or specific volume can be independent of change in one of the others, i.e., change in one may or may not effect a change in others.

Ratio of matrix to magnetic material in the composition can range from about 1:20 (matrix:magnetic) to about 20:1. The ratio can be in the range of from about 1:120 to about 10:1, from about 1:30 to about 5:1, or from about 1:20 to about 1:1. In some embodiments, the ratio is in the range of about 1:20 to about 1:5. In some embodiments, the ratio is around 1:13. In one preferred embodiment, the ratio is in the range from about 1:1 to about 3:1. It is to be understood that ratio can be based on weight, volume and/or moles.

A composition described herein can comprise any amount of matrix and magnetic materials. For example, the composition can comprise 0.1-50% of the matrix material and 10-99% of the magnetic material. In some embodiments, the composition comprises 1-25% of the matrix material and 75-99% of the magnetic material. Preferably, the composition comprises 1-20% of the matrix material and 80-90% of the magnetic material. In some even more preferred embodiments, the composition comprises 1-15% of the matrix material and 85-99% of the magnetic material. In one most preferred embodiment, the composition comprises 5-10% of the matrix material and 90-95% of the magnetic material. The percent values can be based on weight, volume and/or moles. In addition to the matrix and the magnetic materials, the composition can comprise additional substances such as a swelling agent. When an additional substance is present in the composition, matrix and magnetic material percent values are calculated based on the matrix and magnetic material only.

Alternatively, composition can comprise 0.1 %-50% of matrix material, 1-90% of magnetic material and a swelling agent in an amount corresponding to the balance up to 100%. Again percent values can be based on weight, volume and/or moles. In one embodiment, percent values are based on weight. In some embodiments, the composition comprises 0.1 %-25% of matrix material, 5-50% of magnetic material and a swelling agent in an amount corresponding to the balance up to 100%. In some other embodiments, the composition comprises 0.1%-25% of matrix material, 10-50% of magnetic material and a swelling agent in an amount corresponding to the balance up to 100%. In some embodiments, the composition comprises 0.1 %-10% of matrix material, 10-50% of magnetic material and a swelling agent in an amount corresponding to the balance up to 100%. In one embodiment, the composition comprises about 1% matrix, about 13% magnetic material and a swelling agent in an amount corresponding to the balance up to 100%.

The electromagnetic signal generator can include an electromagnet or electrically-polarizable element, or at least one permanent magnet. The electromagnetic signal can be produced at least in part according to a pre-programmed pattern. The electromagnetic signal may have a defined magnetic field strength or spatial orientation, or a defined electric field strength or spatial orientation. In some embodiments, the electromagnetic signal has a defined magnetic field strength. As used herein, the term "magnetic field" refers to magnetic influences which create a local magnetic flux that flows through the composition and can refer to field amplitude, squared-amplitude, or time-averaged squared-amplitude. It is to be understood that magnetic field can be a direct-current (DC) magnetic field or alternating-current (AC) magnetic field. Magnetic field strength can range from about 0.001 Tesla to about 1 Tesla. In some embodiments, magnetic field strength is in the range from about 0.01 Tesla to about 1 Tesla. In some other embodiments, magnetic field strength is in the range from about 0.1 Tesla to about 1 Tesla. Typically, the magnetic field strength is 0.5 Tesla.

In certain embodiments, the composition is macroporous. As used herein, the term "macroporous" refers to the fact that composition comprises macropores. The composition can also comprise micropores. Generally, micropores are pores having a diameter on the order of about 50 Angstroms or less, while macropores are pores having a diameter on the order of about 100 Angstroms or greater. Generally, diameter of a pore is such as to allow free flow of swelling agent and/or a solvent through the pores. Typically, pore diameters of compositions described herein can range from about 0.01 µm to 1000 µm. In some embodiments, the composition comprises pores having mean pore diameter in range from about 150 µm to 1000 µm. In some embodiments, the composition comprises pores having mean pore diameter of 200-750µm. In one preferred embodiment, the composition comprises pores having mean pore diameter of 250-700µm. In another preferred embodiment, the composition comprises pores having mean pore diameter of 500-700µm. In one embodiment, the composition comprises pores having mean pore diameter of around 700 µm.

The changes in porosity, pore size, pore connectivity, swelling agent concentration, and/or specific volume are preferably reversible (i.e., porosity, pore size, pore connectivity, swelling agent concentration, and/or specific volume detectably increases or decreases upon application of the stimuli, and then reverts to its original value, e.g., within 10%, 5%, 2%, 1% or less of the original value, when the stimuli is discontinued). However, it will be recognized that in some applications, reversibility of one or more of porosity, pore size, pore connectivity, swelling agent concentration, and/or specific volume is not essential. The change in specific volume is also referred to as a volume phase transition herein.

It is to be understood that external stimuli can be applied by providing a stimuli that is not present, by holding back a stimuli that is already present, or by changing the amount of a stimuli that is already present.

In some embodiments, in response to an electromagnetic signal, porosity, pore size, pore connectivity, swelling agent concentration, and/or specific volume changes by at least 10%, 15%, 20%, 25% 30%, 40%, 50%, 60%, 70%, 80%, 90%, 95%, 96 %, 97%, 98%, 99% or more relative to the original value. In some embodiments, in response to an electromagnetic signal, porosity, pore size, pore connectivity, swelling agent concentration, and/or specific volume changes by at least 70% or more relative to the original value. Preferably, the porosity, pore size, pore connectivity, swelling agent concentration, and/or specific volume decreases in response to the electromagnetic signal.

As used herein, the term "porosity" means the fractional volume (dimension-less) of the composition that is composed of open space, e.g., pores or other openings. See for example, Coulson J.M., et. al., Chemical Engineering, 1978, volume 2, 3rd Edition, Pergamon Press, 1978, page 126). Generally, in the absence of an external electromagnetic signal, porosity of the composition can range from 0.5 to 0.99. Preferably porosity is in the range of from about 0.75 to about 0.99, more preferably from about 0.8 to about 0.95. Preferably, porosity of the porous material is at least 0.75, more preferably at least 0.8, and most preferably at least 0.9.

In some embodiments, in the presence of an external electromagnetic signal, porosity of the composition can be 50% or less, 40% or less, 30% or less, 25% or less, 20% or less, 15% or less, 10% or less, 5 % or less relative to the porosity of the composition in absence of an external electromagnetic signal

Several methods can be employed to measure porosity, including, direct methods (e.g. determining the bulk volume of the porous sample, and then determining the volume of the skeletal material with no pores (pore volume = total volume - material volume), optical methods (e.g., determining the area of the material versus the area of the pores visible under the microscope, where the areal and volumetric porosities are equal for porous media with random structure), imbibition methods (e.g., immersion of the porous sample, under vacuum, in a fluid that preferentially wets the pores), water saturation method (e.g., pore volume = total volume of water - volume of water left after soaking), water evaporation method (e.g., pore volume in cubic centimeters = weight of saturated sample in grams - weight of dried sample in grams), and gas expansion methods. Methods for measuring porosity of a sample are described in Glasbey, C.A. Horgan, G.W. and Darbyshire, J.F., J. Soil Sci. 1991, 42, 479 - 486.

In some embodiments, the composition is a particle, e.g. a nanoparticle or microparticle. The composition particle size will vary depending on the particular use intended for such a particle. In general, particles can have at least one dimension in the range from about 1000 µm to about 2000 µm. The mass median particle size can generally be from about 1200 to about 1500 µm.

Preparation of compositions described herein does not require a special type of matrix and/or magnetic materials. Any material that comprises a spatial network structure can be used for the matrix. The matrix can comprise materials of synthetic or natural origin (e.g., biopolymers) or a mixture thereof cross-linked by physical and/or chemical interactions. In some embodiments, the matrix is not biodegradable.

Some exemplary matrixes include swellable and non-swellable gels, elastomers, and rubbers. Swellable gels can include hydrogels and organogels. The term "hydrogel" indicates a cross-linked, water insoluble, water containing material. Hydrogels have many desirable properties for biomedical applications. For example, they can be made nontoxic and compatible with tissue, and they are usually highly permeable to water, ions and small molecules.

Gels generally comprise solid, cross-linked polymer networks capable of forming a stable system in equilibrium with an interpenetrating swelling agent. Many gel forming polymers are known in the art. Suitable gels include polymers, copolymers, and blockpolymers based on monomers containing ionizable groups or polymerizable double bonds. Exemplary monomers include, but are not limited to, acrylic acid, methyl methacrylate, methyl acrylic acid, ethyl acrylate, vinyl sulfonic acid, styrene, styrene sulfonic acid (e.g., p-styrene sulfonic acid), maleic acid, butenoic acid, vinyl phosphate, vinyl phosphonate, ethylene, propylene, styrene, vinyl methyl ether, vinyl acetate, vinyl alcohol, acrylonitrile, acrylamide, N-(C₁-C₆ alkyl) acrylamide (such as N-isopropylacrylamide, N-t-butylacrylamide), and the like. Gels are made by homopolymerizing or copolymerizing any of the foregoing monomers. Other suitable gel materials can include, alginate, chitosan, collagen, gelatin, hyaluronate, fibrin, agarose, and derivatives thereof. The gel can be a copolymer as described above into which has been incorporated as one comonomeric component a ligand that connects to, complexes or physically entraps the desired magnetic material.

The gel can be cross-linked to let it take a physically stable form when hydrated or dehydrated. Suitable cross-linking can be provided by incorporating about 0.5 wt. % to about 1.5% wt. % of a cross-linking agent into the gel. Cross-linking can also be provided by incorporating about 0.01 mol % to about 15 mol % of the cross-linking agent in the gel.

Suitable crosslinking agents include compounds whose molecule has a plurality of reactive groups. Such molecular crosslinking agents may be N, N' - methylene-bis acrylamide or divinylbenzene (DVB), ethylene glycol dimethacrylate, divinyl ketone, vinyl methacrylate and divinyl oxalate. Ionic crosslinkage which uses ions such as metallic ions may also be employed. Crosslinkage using electromagnetic waves such as gamma rays is also possible. Cross-linking can also be based on electrostatic interactions, hydrogen bonding, hydrophobic interactions or (micro)crystal formation.

Ionically cross-linkable polymers can be anionic or cationic in nature and include, but are not limited to, carboxylic, sulfate, hydroxyl and amine functionalized polymers. The cross-linking ions used to crosslink the polymers can be anions or cations depending on whether the polymer is anionically or cationically cross-linkable. Appropriate cross-linking ions include, but are not limited to, cations selected from the group consisting of calcium, magnesium, barium, strontium, boron, beryllium, aluminum, iron, copper, cobalt, lead and silver ions. Anions can be selected from, but are not limited to, the group consisting of phosphate, citrate, borate, succinate, maleate, adipate and oxalate ions. More broadly, the anions are derived from polybasic organic or inorganic acids. Preferred cross-linking cations are calcium, iron, and barium ions. The most preferred cross-linking cations are calcium and barium ions. The most preferred cross-linking anion is phosphate. Cross-linking can be carried out by contacting the polymers with a nebulized droplet containing dissolved ions. One of ordinary skill in the art will be able to select appropriate cross-linking agent for the respective hydrogel used in the making of a multi-layer TE construct. For example, the gelation of collagen or alginate occurs in the presence of ionic cross-linker or divalent cations such as Ca²⁺, Ba²⁺ and Sr²⁺.

In some embodiments, the gel comprises a biodegradable polymer selected from the group consisting of polyanhydrides, polyhydroxybutyric acid, polyorthoesters, polysiloxanes, polycaprolactone, poly(lactic-co-glycolic acid), poly(lactic acid), poly(glycolic acid), and copolymers prepared from the monomers of these polymers.

Suitable polymers which can be used in the present invention include, but are not limited to, one or a mixture of polymers selected from the group consisting of glycosaminoglycan, silk, fibrin, MATRIGEL®, poly-ethyleneglycol (PEG), polyhydroxy ethyl methacrylate, polyvinyl alcohol, polyacrylamide, poly (N-vinyl pyrolidone), poly glycolic acid (PGA), poly lactic-co-glycolic acid (PLGA), poly e-carpolactone (PCL), polyethylene oxide, poly propylene fumarate (PPF), poly acrylic acid (PAA), hydrolysed polyacrylonitrile, polymethacrylic acid, polyethylene amine, alginic acid, pectinic acid, carboxy methyl cellulose, hyaluronic acid, heparin, heparin sulfate, chitosan, carboxymethyl chitosan, chitin, pullulan, gellan, xanthan, collagen, gelatin, carboxymethyl starch, carboxymethyl dextran, chondroitin sulfate, cationic guar, cationic starch as well as salts and esters thereof. Polymers listed above which are not ionically cross-linkable are used in blends with polymers which are ionically cross-linkable. In some embodiments polymer is not gelatin.

Other preferred polymers include esters of alginic, pectinic or hyaluronic acid and C2 to C4 polyalkylene glycols, e.g. propylene glycol, as well as blends containing 1 to 99 wt % of alginic, pectinic or hyaluronic acid with 99 to 1 wt % polyacrylic acid, polymethacrylic acid or polyvinylalcohol. Preferred blends comprise alginic acid and polyvinylalcohol. Examples of mixtures include, but are not limited to, a blend of polyvinyl alcohol (PVA) and sodium alginate and propyleneglycol alginate.

In some embodiments, gel is alginate, collagen, or agarose. When the gel is alginate, the alginate can be an oxidized alginate. Generally alginate can be fully oxidized or partially oxidized. When partially oxidized, oxidation of alginate can range from about 0.5% to about 30%, from about 0.75% to about 20%, from about 1% to about 10%, and/or from about 1% to about 5%. Without wishing to be bound by theory, oxidized alginate can be degraded via hydrolysis.

As used herein, the term "magnetic material" refers to a material or substance that is influenced by a magnetic field, i.e. relative permeability (µᵣ) of the material is greater than unity. Such magnetic materials are intended to include those which are referred to as ferromagnetic, diamagnetic, paramagnetic, and superparamagnetic. As is the conventional understanding given that term, superparamagnetic materials exhibit magnetic properties only when in an externally applied magnetic field, and otherwise exhibit essentially no magnetic properties; and their total magnetism is greater than the sum of that of the individual particles considered separately. If the particle size of the magnetic material is sufficiently small, the magnetic material will most likely be superparamagnetic.

The magnetic properties of the composition are greatly influenced by the saturation magnetization, size, and concentration of magnetic material, as well as the strength of the external magnetic field.

The magnetic material can be any molecule, composition, particle, or substance, that exhibits magnetic properties when incorporated into the matrix. The magnetic materials can be selected from the group of elements having atomic numbers 21-29, 42, 44, and 57-70, elements having atomic numbers 24-29 or 62-69 being especially preferred. Preferably, a magnetic material is selected from the group including but not limited to, rare earth metals (such as gadolinium, terbium, dysprosium, holmium, erbium and europium), transient metals (such as iron, nickel, cobalt, magnesium chromium and copper), noble metals (such as rhodium, palladium), their oxides, compositions, combinations, solid dispersions, and alloys.

In some embodiments, the magnetic material is selected from the group consisting of maghemite (Fe₂O₃), magnetite (Fe₃O₄), strontium ferrite, samarium-cobalt, neodymium-iron-boron (NIB), lodestone, pyrrhotite, BaFe₁₂O₁₉, Alnico magnet alloy, transfer salts of decamethylmetallocenes with 7,7,8,8-tetracyano-p-quinodimethane (TCNQ) or tetracyanoethenide (TCNE) (such as [Fe(Cp*)₂]⁺[TCNE]⁻, [Fe(Cp*)₂]⁺[TCNQ]⁻, [Cr(Cp*)₂]⁺[TCNE]⁻, [Cr(Cp*)₂]⁺[TCNQ]⁻, [Mn(Cp*)₂]⁺[TCNE]⁻, and [Mn(Cp*)₂]⁺[TCNQ]⁻), hexylammonium trichlorocuprate(II) (CᵤCl₃(C₆H₁₁NH₃), Fe based amorphous magnetic powders, and combinations thereof. In some embodiments, the composition of the invention comprises two or more, e.g., two, three, four, five, different magnetic materials.

Exemplary Fe based amorphous magnetic powders are described in U.S. Pat. App. Pub. No. 2009/0232693.

In some embodiments, magnetic material is a particle, e.g. a magnetic nanoparticle, magnetic microparticle. Depending on the size, porosity or pore size of matrix, magnetic particles can rang in size from 1nm to 1000 µms. Preferably magnetic particles are about 1nm to 500 nm in size. In some embodiments, magnetic particle is a magnetic nanoparticle of size about 300nm. Magnetic nanoparticles are a class of nanoparticle which can be manipulated using magnetic field. Such particles commonly consist of magnetic elements such as iron, nickel and cobalt and their chemical compounds. Magnetic nano-particles are well known and methods for their synthesis are described in the art, for example, in U.S. Pat. No. 6,878,445; No. 5,543,158; No. 5,578,325; No. 6,676,729; No. 6,045,925 and No. 7,462,446, and U.S. Pat. Pub. No. 2005/0025971; No. 2005/0200438; No. 2005/0201941; No. 2005/0271745; No. 2006/0228551; No. 2006/0233712; No. 2007/01666232 and No. 2007/0264199.

The magnetic material should be sufficiently immobilized in the matrix so that during any application of a magnetic field it cannot be removed therefrom by dissolution or chemical reaction that would be encountered, even as a result of a change in the porosity, pore size, pore connectivity, swelling agent concentration, and/or specific volume of the matrix. Thus, the magnetic material can be simply physically entrapped within the matrix, or it can be chemically bound into the matrix or complexed, encased in, or physically immobilized by an intermediate ligand which is in turn, chemically bound into the matrix. It is to be understood that physical immobilization includes chelation.

Without limitations, the magnetic material can be distributed homogeneously or heterogeneously within the matrix material. As used herein, a homogenous distribution of the magnetic material means the magnetic material is randomly and/or uniformly distributed within the matrix material. A heterogeneous distribution of the magnetic material means that the magnetic material is not randomly and/or uniformly distributed within the matrix material. Thus, a heterogeneous distribution includes magnetic material distributed in one or more patterns in the matrix materials. Without wishing to be bound by theory, a non-random distribution allows different regions of the composition to respond distinctly to a magnetic field. For example, such differential response can be used to release agents encapsulated in the different regions under different conditions and/or independently of relase from other regions. Alternatively, or additionally, a composition can comprise one or more regions with release controlled by a magnetic field and one or more region that do not require an external trigger for releasing an encapsulated agent from the composition.

Compositions of the invention can be prepared using methods known in the art and easily adapted by one of skill in the art. For example, magnetic material can be bound to the matrix by carrying out the polymerization which forms the matrix in the presence of chelate-forming groups and then reacting this intermediate with an excess of the magnetic material in an aqueous solution. If desired, a bridging group, e.g. a linker, can be introduced between the chelate-forming groups and the matrix backbone, e.g. in a manner known per se.

Alternatively, the magnetic material can be present in cavities within the matrix, in the form of an insoluble or sparingly soluble substance or composition. The incorporation of the magnetic material within the matrix can be achieved in several ways.

In one method dry or incompletely swollen matrix may be swelled in an appropriate solution comprising a salt of a metal, for instance chloride and/or sulfate of said metal, whereafter the matrix is dried. The matrix is then swelled again in a solution, of a substance which is capable of precipitating the metal in the form of an insoluble or sparingly soluble magnetic material, compound or complex. For instance the precipitating substance may be a soluble phosphate, such as sodium phosphate, when the phosphate of the metal is insoluble or sparingly soluble in the medium in which the matrix is swelled. Alternatively, the precipitating substance may be an alkali metal hydroxide when the hydroxide of the metal is insoluble or sparingly soluble in the medium in which the matrix is swelled.

As used herein, the term "magnetic metal" refers to any metal that exhibits magnetic properties when it is incorporated in the matrix. The magnetic metal may or may not exhibit magnetic properties while it is not incorporated in the matrix. As used herein, magnetic metals includes ions, salts, oxides or nitrides of the metal.

According to another method, dry or incompletely swollen matrix material is swelled in a solution comprising a solvent in which the matrix material swells, e.g. water or dimethylsulfoxide, and one or more reagents of which at least one comprises the magnetic material in a suitable chemical form, and which reagents (optionally in contact with the matrix) produce by a chemical reaction (which may involve the matrix), for example a redox process, the magnetic material in elemental state or in the state of an insoluble or sparingly soluble chemical compound containing the magnetic material, said magnetic material being finely dispersed in the matrix.

According to another method the matrix is prepared by a process involving a cross-linking reaction carried out in a medium in which magnetic material or a complex thereof is dispersed, the magnetic material or complex being insoluble or sparingly soluble in said medium. Thus, the magnetic material or complex will become entrapped in a dispersed form in cavities formed in the three-dimensional network of the matrix. Where the magnetic material is incorporated as a complex, this is preferably a chelate complex which is insoluble or sparingly soluble in aqueous media.

Methods for preparing ferrogels have been described, for example, in Sahiner, N., Colloid Polym. Sci. 2006, 285, 283; Sauzeddle, F. Elaissari, A. and Picho, C., Colloid Polym. Sci. 1999, 277, 846; Gu, S. Shiratori, T. and Konno, M., Colloid Polym. Sci. 2003, 281,1076; Zhang, J. et al., Adv. Mater. 2002, 14, 1756; and Caykara, T. Yörök, D. and Demirci, S., J. App. Polym. Sci. 2009, 112, 800.

Once matrix comprising magnetic material has been prepared, macropores can be introduced by freezing said matrix at temperature ranging from about -10°C to about -180°C and lyophilizing the matrix. In some embodiments, freezing temperature is about -15°C to about -25°C. For example, to prepare compositions comprising pores of size about 700µm, a freezing temperature of about -20°C is preferred. Lyophilized composition is then swelled in the appropriate swelling agent. Without wishing to be bound by theory, freezing temperature effects porosity and pore size of the resultant composition.

In some embodiments, the composition of the invention has an elastic modulus in the range between 10⁻³ and 10⁸ kPa. In some further embodiments, of this, the composition has an elastic modulus in the range between 10⁻³ and 10³ kPa. As used herein, the term "elastic modulus" refers to an object or substance's tendency to be deformed elastically (i.e., non-permanently) when a force is applied to it. Generally, the elastic modulus of an object is defined as the slope of its stress-strain curve in the elastic deformation region. Specifying how stress and strain are to be measured, including directions, allows for many types of elastic moduli to be defined. Young's modulus (E) describes tensile elasticity, or the tendency of an object to deform along an axis when opposing forces are applied along that axis; it is defined as the ratio of tensile stress to tensile strain. It is often referred to simply as the elastic modulus. The shear modulus or modulus of rigidity (G or µ) describes an object's tendency to shear (the deformation of shape at constant volume) when acted upon by opposing forces; it is defined as shear stress over shear strain. The shear modulus is part of the derivation of viscosity. The bulk modulus (K) describes volumetric elasticity, or the tendency of an object to deform in all directions when uniformly loaded in all directions; it is defined as volumetric stress over volumetric strain, and is the inverse of compressibility. The bulk modulus is an extension of Young's modulus to three dimensions. Three other elastic moduli are Poisson's ratio, Lamé's first parameter, and P-wave modulus.

In some embodiments, the composition further comprises a compound selected from the group consisting of small organic or inorganic molecules; saccharines; oligosaccharides; polysaccharides; biological macromolecules, e.g., peptides, proteins, and peptide analogs and derivatives; peptidomimetics; nucleic acids, e.g., oligonucleotides, antisense oligonucleotides, siRNAs, shRNAs, ribozymes, aptamers, microRNAs, pre-microRNAs, plasmid DNA, etc...; nucleic acid analogs and derivatives; an extract made from biological materials such as bacteria, plants, fungi, or animal cells; animal tissues; naturally occurring or synthetic compositions; and any combinations thereof. In some embodiments, the test compound is a small molecule.

As used herein, the term "small molecule" can refer to compounds that are "natural product-like," however, the term "small molecule" is not limited to "natural product-like" compounds. Rather, a small molecule is typically characterized in that it contains several carbon-carbon bonds, and has a molecular weight of less than 5000 Daltons (5 kD), preferably less than 3 kD, still more preferably less than 2 kD, and most preferably less than 1 kD. In some cases it is preferred that a small molecule have a molecular mass equal to or less than 700 Daltons.

As used herein, the term "peptide" is used in its broadest sense to refer to compounds containing amino acids, amino acid equivalents or other non-amino groups, while still retaining the desired functional activity of a peptide. Peptide equivalents can differ from conventional peptides by the replacement of one or more amino acids with related organic acids (such as PABA), amino acids or the like or the substitution or modification of side chains or functional groups. The peptides can be linear or cyclic. A peptide can be modified to include one or more of D-amino acids, beta-amino acids, chemically modified amino acids, naturally occurring non-proteogenic amino acids, rare amino acids, and chemically synthesized compounds that have properties known in the art to be characteristic of an amino acid.

As used herein, the term "nucleic acid" or "oligonucleotide" or grammatical equivalents herein means at least two nucleotides, including analogs or derivatives thereof, that are covalently linked together. Exemplary oligonucleotides include, but are not limited to, single-stranded and double-stranded siRNAs and other RNA interference reagents (RNAi agents or iRNA agents), shRNA (short hairpin RNAs), antisense oligonucleotides, aptamers, ribozymes, and microRNAs (miRNAs). The nucleic acids can be single stranded or double stranded. The nucleic acid can be DNA, RNA or a hybrid, where the nucleic acid contains any combination of deoxyribo- and ribo-nucleotides, and any combination of uracil, adenine, thymine, cytosine and guanine. The nucleic acids can comprise one or more backbone modifications, e.g., phosphoramide (Beaucage et al., Tetrahedron 49(10):1925 (1993) and references therein; Letsinger, J. Org. Chem. 35:3800 (1970)), phosphorothioate, phosphorodithioate, O-methylphophoroamidite linkages (see Eckstein, Oligonucleotides and Analogues: A Practical Approach, Oxford University Press), or peptide nucleic acid linkages (see Egholm, J. Am. Chem. Soc. 114:1895 (1992); Meier et al., Chem. Int. Ed. Engl. 31:1008 (1992); and Nielsen, Nature, 365:566 (1993). The nucleic acids can also include modifications to nucleobase and/or sugar moietites of nucleotides. Examplary sugar modifications at the sugar moiety include replacement of 2'-OH with halogens (e.g., fluoro), O-mehtyl, O-methoxyethyl, NH₂, SH and S-methyl.

As used herein, the term "polysaccharide" refers to macromolecular carbohydrates whose molecule consists of a large number of monosaccharide molecules which are joined to one another by glycosidic linkage. The term polysaccharide is also intended to embrace an oligosaccharide. The polysaccharide can be homopolysaccharides or heteropolysaccharides. Whereas the homopolysaccharides contain only one kind of unit, the heteropolysaccharides consist of monomer units of different kinds.

In some embodiments, the composition further comprises a bioactive agent. As used herein, "bioactive agents" or "bioactive materials" refer to naturally occurring biological materials, for example, extracellular matrix materials such as fibronectin, vitronection, and laminin; cytokines; growth factors and differentiation factors; nucleic acids; proteins; peptides; and cells. "Bioactive agents" also refer to artificially synthesized materials, molecules or compounds that have a biological effect on a biological cell, tissue or organ.

Suitable growth factors and cytokines include, but are not limited, to stem cell factor (SCF), granulocyte-colony stimulating factor (G-CSF), granulocyte-macrophage stimulating factor (GM-CSF), stromal cell-derived factor-1, steel factor, VEGF, TGFβ, platelet derived growth factor (PDGF), angiopoeitins (Ang), epidermal growth factor (EGF), bFGF, HNF, NGF, bone morphogenic protein (BMP), fibroblast growth factor (FGF), hepatocye growth factor, insulin-like growth factor (IGF-1), interleukin (IL)-3, IL-1α, IL-1β, IL-6, IL-7, IL-8, IL-11, and IL-13, colony-stimulating factors, thrombopoietin, erythropoietin, fit3-ligand, and tumor necrosis factor α (TNFα□□)□. Other examples are described in Dijke et al., "Growth Factors for Wound Healing", Bio/Technology, 7:793-798 (1989); Mulder GD, Haberer PA, Jeter KF, eds. Clinicians' Pocket Guide to Chronic Wound Repair. 4th ed. Springhouse, PA: Springhouse Corporation; 1998:85; Ziegler T.R., Pierce, G.F., and Herndon, D.N., 1997, International Symposium on Growth Factors and Wound Healing: Basic Science & Potential Clinical Applications (Boston, 1995, Serono Symposia USA), Publisher: Springer Verlag.

In some embodiments, suitable bioactive agents include, but are not limited to, therapeutic agents. As used herein, the term "therapeutic agent" refers to a substance used in the diagnosis, treatment, or prevention of a disease. Any therapeutic agent known to those of ordinary skill in the art to be of benefit in the diagnosis, treatment or prevention of a disease is contemplated as a therapeutic agent in the context of the present invention. Therapeutic agents include pharmaceutically active compounds, hormones, growth factors, enzymes, DNA, plasmid DNA, RNA, siRNA, viruses, proteins, lipids, pro-inflammatory molecules, antibodies, antibiotics, anti-inflammatory agents, anti-sense nucleotides and transforming nucleic acids or combinations thereof. Any of the therapeutic agents may be combined to the extent such combination is biologically compatible.

Exemplary therapeutic agents include, but are not limited to, those found in Harrison's Principles of Internal Medicine, 13th Edition, Eds. T.R. Harrison et al. McGraw-Hill N.Y., NY; Physicians Desk Reference, 50th Edition, 1997, Oradell New Jersey, Medical Economics Co.; Pharmacological Basis of Therapeutics, 8th Edition, Goodman and Gilman, 1990; United States Pharmacopeia, The National Formulary, USP XII NF XVII, 1990; current edition of Goodman and Oilman's The Pharmacological Basis of Therapeutics; and current edition of *The Merck Index.*

Examples of therapeutic agents which may be incorporated in the composition, include, but are not limited to, narcotic analgesic drugs; salts of gold; corticosteroids; hormones; antimalarial drugs; indole derivatives; pharmaceuticals for arthritis treatment; antibiotics, including Tetracyclines, Penicillin, Streptomycin and Aureomycin; antihelmintic and canine distemper drugs, applied to domestic animals and large cattle, such, as, for example, phenothiazine; drugs based on sulfur, such, as sulfioxazole; antitumor drugs; pharmaceuticals supervising addictions, such as agents controlling alcohol addiction and agents controlling tobacco addiction; antagonists of drug addiction, such, as methadone; weight controlling drugs; thyroid gland controlling drugs; analgesics; drugs controlling fertilization or contraception hormones; amphetamines; antihypertensive drugs; antiinflammatories agents; antitussives; sedatives; neuromuscular relaxants; antiepileptic drugs; antidepressants; antidisrhythmic drugs; vasodilating drugs; antihypertensive diuretics; antidiabetic agents; anticoagulants; antituberculous agents; antipsychotic agents; hormones and peptides. It is understood that above list is not full and simply represents the wide diversification of therapeutic agents that may be included in the compositions. In some embodiments, therapeutic agent is Mitoxantrone, protein (e.g. VEGF) or plasmid DNA.

The amount of therapeutic agent distributed in a composition depends on various factors including, for example, specific agent; function which it should carry out; required period of time for release of a the agent; quantity to be administered. Generally, dosage of a therapeutic agent i.e. amount of therapeutic agent in composition, is selected from the range about from 0.001% (w/w) up to 95% (w/w), preferably, from about 5% (w/w) to about 75% (w/w), and, most preferably, from about 10% (w/w) to about 60% (w/w).

In some embodiments, the composition comprises a cell, e.g. a biological cell. One way to incorporate cells into the composition is by reswelling a dried or partially dried composition of the invention in an aqueous solution comprising the cells to be incorporated. The aqueous solution can comprise from about 10⁴ to about 10⁸ cells/ml. In some embodiments, aqueous solution comprises from about 10⁴ to about 10⁶ cells/ml. In one preferred embodiment, aqueous solution comprises about 5x10⁵ cells/ml.

In some embodiments, the composition comprises more that one cell type. This can be accomplished by having two or more different cell types in aqueous solution used for swelling. When two or more different cell types are to be incorporated into the composition, total number of cells in the aqueous solution ranges from about 10⁴ to about 10⁸ cells/ml, about 10⁴ to about 10⁶ cells/ml, or about 10⁵ cells/ml.

Cells amenable to be incorporated into the composition include, but are not limited to, stem cells (embryonic stem cells, mesenchymal stem cells, bone-marrow derived stem cells and hematopoietic stem cells), chrondrocytes progenitor cells, pancreatic progenitor cells, myoblasts, fibroblasts, keratinocytes, neuronal cells, glial cells, astrocytes, pre-adipocytes, adipocytes, vascular endothelial cells, hair follicular stem cells, endothelial progenitor cells, mesenchymal cells, neural stem cells and smooth muscle progenitor cells.

In some embodiments, the cell is a genetically modified cell. A cell can be genetically modified to express and secrete a desired compound, e.g. a bioactive agent, a growth factor, differentiation factor, cytokines, and the like. Methods of genetically modifying cells for expressing and secreting compounds of interest are known in the art and easily adaptable by one of skill in the art.

Differentiated cells that have been reprogrammed into stem cells can also be used. For example, human skin cells reprogrammed into embryonic stem cells by the transduction of Oct3/4, Sox2, c-Myc and Klf4 (Junying Yu, et. al., Science, 2007, 318, 1917-1920 and Takahashi K. et. al., Cell, 2007, 131, 1-12).

Cells useful for incorporation into the composition can come from any source, for example human, rat or mouse. Human cells include, but are not limited to, human cardiac myocytes-adult (HCMa), human dermal fibroblasts-fetal (HDF-f), human epidermal keratinocytes (HEK), human mesenchymal stem cells-bone marrow, human umbilical mesenchymal stem cells, human hair follicular inner root sheath cells, human umbilical vein endothelial cells (HUVEC), and human umbilical vein smooth muscle cells (HUVSMC), human endothelial progenitor cells, human myoblasts, human capillary endothelial cells, and human neural stem cells.

Exemplary rat and mouse cells include, but not limited to, RN-h (rat neurons-hippocampal), RN-c (rat neurons-cortical), RA (rat astrocytes), rat dorsal root ganglion cells, rat neuroprogenitor cells, mouse embryonic stem cells (mESC) mouse neural precursor cells, mouse pancreatic progenitor cells mouse mesenchymal cells and mouse endodermal cells.

In some embodiments, tissue culture cell lines can be used in the compositions described herein. Examples of cell lines include, but are not limited to, C166 cells (embryonic day 12 mouse yolk), C6 glioma Cell line, HL1 (cardiac muscle cell line), AML12 (nontransforming hepatocytes), HeLa cells(cervical cancer cell line) and Chinese Hamster Ovary cells (CHO cells).

An ordinary skill artisan in the art can locate, isolate and expand such cells. In addition, the basic principles of cell culture and methods of locating, isolation and expansion and preparing cells for tissue engineering are described in "Culture of Cells for Tissue Engineering" Editor(s): Gordana Vunjak-Novakovic, R. Ian Freshney, 2006 John Wiley & Sons, Inc., and Heath C. A., Trends in Biotechnology, 2000, 18, 17-19.

The bioactive agent can be covalently linked to the matrix through a linker. The linker can be a cleavable linker or non-cleavable linker, depending on the application. As used herein, a "cleavable linker" refers to linkers that are capable of cleavage under various conditions. Conditions suitable for cleavage can include, but are not limited to, pH, UV irradiation, enzymatic activity, temperature, hydrolysis, elimination and substitution reactions, redox reactions, and thermodynamic properties of the linkage. In many cases, the intended nature of the conjugation or coupling interaction, or the desired biological effect, will determine the choice of linker group.

In some embodiments, the bioactive agent is bound to the matrix by a hydrolyzable bond. In some embodiments, the cell or the bioactive agent has a mean free path in the composition that is shorter than the mean free path of the cell or the bioactive agent in water.

In some embodiments, the matrix is functionalized with binding molecules that binds with a bioactive molecule. These binding molecules are also referred to as affinity molecules herein. The binding molecule can be bound covalently (directly or through a linker) or non-covalently to the matrix. The binding molecule can be selected such that it can bind to any part of bioactive molecule that is accessible.

As used herein, the term "binding molecule" or "affinity molecule" refers to any molecule that is capable of binding a bioactive molecule. Representative examples of affinity molecules include, but are not limited to, antibodies, antigens, lectins, proteins, peptides, nucleic acids (DNA, RNA, PNA and nucleic acids that are mixtures thereof or that include nucleotide derivatives or analogs); receptor molecules, such as the insulin receptor; ligands for receptors (e.g., insulin for the insulin receptor); and biological, chemical or other molecules that have affinity for another molecule, such as biotin and avidin. The binding molecules need not comprise an entire naturally occurring molecule but may consist of only a portion, fragment or subunit of a naturally or non-naturally occurring molecule, as for example the Fab fragment of an antibody.

Nucleic acid based binding molecules include aptamers. As used herein, the term "aptamer" means a single-stranded, partially single-stranded, partially double-stranded or double-stranded nucleotide sequence capable of specifically recognizing a selected non-oligonucleotide molecule or group of molecules by a mechanism other than Watson-Crick base pairing or triplex formation. Aptamers can include, without limitation, defined sequence segments and sequences comprising nucleotides, ribonucleotides, deoxyribonucleotides, nucleotide analogs, modified nucleotides and nucleotides comprising backbone modifications, branchpoints and nonnucleotide residues, groups or bridges. Methods for selecting aptamers for binding to a molecule are widely known in the art and easily accessible to one of ordinary skill in the art.

In some embodiments of the aspects described herein, the binding molecules are polyclonal and/or monoclonal antibodies and antigen-binding derivatives or fragments thereof. Well-known antigen binding fragments include, for example, single domain antibodies (dAbs; which consist essentially of single VL or VH antibody domains), Fv fragment, including single chain Fv fragment (scFv), Fab fragment, and F(ab')2 fragment. Methods for the construction of such antibody molecules are well known in the art. Accordingly, as used herein, the term "antibody" refers to an intact immunoglobulin or to a monoclonal or polyclonal antigen-binding fragment with the Fc (crystallizable fragment) region or FcRn binding fragment of the Fc region. Antigen-binding fragments may be produced by recombinant DNA techniques or by enzymatic or chemical cleavage of intact antibodies. "Antigen-binding fragments" include, inter alia, Fab, Fab', F(ab')2, Fv, dAb, and complementarity determining region (CDR) fragments, single-chain antibodies (scFv), single domain antibodies, chimeric antibodies, diabodies and polypeptides that contain at least a portion of an immunoglobulin that is sufficient to confer specific antigen binding to the polypeptide. The terms Fab, Fc, pFc', F(ab') 2 and Fv are employed with standard immunological meanings (Klein, Immunology (John Wiley, New York, N.Y., 1982); Clark, W. R. (1986) The Experimental Foundations of Modern Immunology (Wiley & Sons, Inc., New York); and Roitt, I. (1991) Essential Immunology, 7th Ed., (Blackwell Scientific Publications, Oxford)). Antibodies or antigen-binding fragments specific for various antigens are available commercially from vendors such as R&D Systems, BD Biosciences, e-Biosciences and Miltenyi, or can be raised against these cell-surface markers by methods known to those skilled in the art.

In some embodiments, the binding molecule binds with a cell. Without wishing to be bound by theory, a molecule that binds with a cell can do so by binding with a cell-surface marker or a cell-surface molecule. These binding molecules that bind with a cell are also referred to as cell binding molecules. In some further embodiments, the binding molecule binds with a cell-surface marker but does not cause initiation of downstream signaling event mediated by that cell-surface marker. Binding molecules specific for cell-surface molecules include, but are not limited to, antibodies or fragments thereof, natural or recombinant ligands, small molecules, nucleic acids and analogues thereof, intrabodies, aptamers, lectins, and other proteins or peptides.

As used herein, a "cell-surface marker" refers to any molecule that is present on the outer surface of a cell. Some molecules that are normally not found on the cell-surface can be engineered by recombinant techniques to be expressed on the surface of a cell. Many naturally occurring cell-surface markers present on mammalian cells are termed "CD" or "cluster of differentiation" molecules. Cell-surface markers often provide antigenic determinants to which antibodies can bind to.

Accordingly, as defined herein, a "binding molecule specific for a cell-surface marker" refers to any molecule that can selectively react with or bind to that cell-surface marker, but has little or no detectable reactivity to another cell-surface marker or antigen. Without wishing to be bound by theory, affinity molecules specific for cell-surface markers generally recognize unique structural features of the markers. In some embodiments of the aspects described herein, the affinity molecules specific for cell-surface markers are polyclonal and/or monoclonal antibodies and antigen-binding derivatives or fragments thereof.

In some embodiments, the cell binding molecule is a ligand that binds to a receptor on the surface of a cell. Such a ligand can be a naturally occurring molecule, a fragment thereof or a synthetic molecule or fragment thereof. In some embodiments, the ligand is non-natural molecule selected for binding with a target cell. High throughput methods for selecting non-natural cell binding ligands are known in the art and easily available to one of skill in the art. See for example, Anderson, et al., Biomaterial microarrays: rapid, microscale screening of polymer-cell interaction. Biomaterials (2005) 26:4892-4897; Anderson, et al., Nanoliter-scale synthesis of arrayed biomaterials and application to human embryonic stem cells. Nature Biotechnology (2004) 22:863-866; Orner, et al., Arrays for the combinatorial exploration of cell adhesion. Journal of the American Chemical Society (2004) 126:10808-10809; Falsey, et al., Peptide and small molecule microarray for high throughput cell adhesion and functional assays. Bioconjugate Chemistry (2001) 12:346-353; Liu, et al., Biomacromolecules (2001) 2(2): 362-368; and Taurniare, et al., Chem. Comm. (2006): 2118-2120.

In some embodiments, the cell binding molecule is an integrin-binding peptide. In some embodiments, the integrin-binding peptide comprises the amino acid sequence Arg-Gly-Asp (RGD). In some embodiments, the cell binding molecule is a peptide comprising the amino acid sequence (Gly)₄-Arg-Gly-Asp-Ala-Ser-Ser-Lys-Tyr (SEQ ID NO: 1).

A composition described herein can be administered to a subject by any appropriate route known in the art including, but not limited to, oral or parenteral routes, including intravenous, intramuscular, subcutaneous, transdermal, airway (aerosol), pulmonary, nasal, rectal, and topical (including buccal and sublingual) administration. As used herein, the term "administer" refers to the placement of a composition into a subject by a method or route which results in at least partial localization of the composition at a desired site such that desired effect is produced.

Exemplary modes of administration include, but are not limited to, injection, infusion, instillation, inhalation, or ingestion. "Injection" includes, without limitation, intravenous, intramuscular, intraarterial, intrathecal, intraventricular, intracapsular, intraorbital, intracardiac, intradermal, intraperitoneal, transtracheal, subcutaneous, subcuticular, intraarticular, sub capsular, subarachnoid, intraspinal, intracerebro spinal, and intrasternal injection and infusion. In some embodiments, the compositions are administered by intravenous infusion or injection.

For administration to a subject, composition comprising a bioactive agent can be formulated together with one or more pharmaceutically acceptable carriers (additives) and/or diluents. The pharmaceutical compositions of the present invention can be specially formulated for administration in solid or liquid form, including those adapted for the following: (1) oral administration, for example, drenches (aqueous or non-aqueous solutions or suspensions), lozenges, dragees, capsules, pills, tablets (e.g., those targeted for buccal, sublingual, and systemic absorption), boluses, powders, granules, pastes for application to the tongue; (2) parenteral administration, for example, by subcutaneous, intramuscular, intravenous or epidural injection as, for example, a sterile solution or suspension, or sustained-release formulation; (3) topical application, for example, as a cream, ointment, or a controlled-release patch or spray applied to the skin; (4) intravaginally or intrarectally, for example, as a pessary, cream or foam; (5) sublingually; (6) ocularly; (7) transdermally; (8) transmucosally; or (9) nasally.

The composition comprising the bioactive agent can be delivered to an *in vivo* locus. Exemplary *in vivo* loci include, but are not limited to site of a wound, trauma or disease. The composition can be delivered to the *in vivo* locus by, for example, implanting the compositions into a subject.

Compositions that are to be implanted can additionally include one or more additives. Additives may be resolving (biodegradable) polymers, mannitol, starch sugar, inosite, sorbitol, glucose, lactose, saccharose, sodium chloride, calcium chloride, amino acids, magnesium chloride, citric acid, acetic acid, hydroxyl-butanedioic acid, phosphoric acid, glucuronic acid, gluconic acid, poly-sorbitol, sodium acetate, sodium citrate, sodium phosphate, zinc stearate, aluminium stearate, magnesium stearate, sodium carbonate, sodium bicarbonate, sodium hydroxide, polyvinylpyrolidones, polyethylene glycols, carboxymethyl celluloses, methyl celluloses, starch or their mixtures.

The implant can have virtually any regular or irregular shape including, but not limited to, spheroid, cubic, polyhedron, prism, cylinder, rod, disc, or other geometric shape.

Accordingly, in some embodiments, the implant is of cylindrical form from about 0.5 to about 10 mm in diameter and from about 0.5 to about 10 cm in length. Preferably, its diameter is from about 1 to about 5 mm and length from about 1 to about 5 cm.

In some embodiments, the implant is of spherical form. When the implant is in a spherical form, its diameter can range from about 0.5 to about 50 mm in diameter. In some embodiments, a spherical implant's diameter is from about 5 to about 30 mm. Preferably the diameter is from about 10 to about 25 mm.

### Controlled drug release

Also described herein is an on-demand method for controlling the release of a bioactive agent, the method comprising: (a) providing or administering to a subject a composition described herein, wherein the composition contains the bioactive agent; and (b) inducing a change in porosity, pore size, pore connectivity, swelling agent concentration, and/or specific volume of the composition via a magnetic field to control the release of the bioactive agent. As used herein, the term "on-demand" refers to the operator control over the release of bioactive agent from the composition.

Release of the bioactive agent can be achieved in a pulsatile manner by repeated application of external stimuli. This ability for on-demand pulsatile release of bioactive agents is useful in a variety of settings, including immunizations, which typically provide an initial immunization, followed by distinct booster doses at later times. Moreover, repeated administration of well-defined doses of allergen, in the absence of immunostimulatory molecules, from such a polymer matrix may be useful for inducing tolerance. It also may be advantageous to use an external stimuli to deliver bioactive agents in a pulsatile manner so as to time the delivery to coincide with a particular biological event (e.g. the circadian rhythm) in order to maximize the effectiveness of the bioactive agent (e.g. against tumors).

In some cases, the composition can release the bioactive agent continuously, and application of a stimuli allows release to of the bioactive agent to be decreased or stopped in emergent clinical situations. For example, an implantable composition can be designed to hold a chemotherapeutic therapeutic agent. Without the presence of an applied magnetic field, the therapeutic agent will continuously disperse out from the composition, however, in the presence of an applied magnetic field, rate of dispersion will be lowered. This can be useful, for example, when there is a need to hold chemotherapeutic therapeutic agents with immunodepressant side-effects while treating infections.

In another example, an implantable composition can be designed to hold an agent such that there is little or no release of the agent in the absence of a stimuli. Without the presence of an applied magnetic field, little or no agent will disperse out from the composition, however, in the presence of an applied magnetic field, rate of dispersion will be increased. This can be useful, for example, when there is a need to hold release of an agent from the composition after placement in a subject. The agent could then be released at a required and/or suitable time by applying the magnetic field.

### Other applications of macroporous gels

Due to its large deformation, fast response, reversible motion and biocompatiblility, the magnetic gels can be used in a physiological environment as a bioactuator. For example, it may be used as a magnetic field controlled artificial muscle.

A significant amount of solvent flows into/out of the magnetic gel during its deformation. Therefore, one may use magnetic field to drive the magnetic gel to act as a pump in microfluidic chips. Further, the reversible deformation of the magnetic gel may also be used as valves in microfluidic chips to open or close microfluidic channels.

Deformation sensitive pigments may also be added into the magnetic gel (Lu Y.F. et al., Nature (2001), 410:19). A magnetic field induces the deformation of the gel, as well as the pigments encapsulated in it. Therefore, the pigments may change color or florescence in response to magnetic field.

Owing to fast response times and substantial specific volume changes in response to an external stimuli, compositions described herein can be employed for numerous applications, particularly in medicine, pharmaceutics, drug-delivery, biosensors, bio-actuators, optical devices, valves and pumps for microfluidics, separation and membranes, purifications, and enzyme and cell immobilization. See for example, Qiu, Y. and Park, K. Adv Drug Delivery Rev (2001), 53: 321-339; Miyata, T. Urgami, T. and Nakamae, K. Adv. Drug Delivery Rev. (2002), 54: 79-98; Bckiari, V. et al., Langmuir (2004), 20: 7972; Lopes, D. Cendoya, L. and Mijangos, C. Macromol Symp (2001), 166:173; Lao, L. and Ramanujan, V.R. J Mater Sci Mater Med (2004), 15: 1061; Porter, J. and Pickup, R.W.J. Microbiol Methods (1998), 33: 221; Xie, X. et al., J Magn Mater (2004), 227: 16 and Gupta, P.K. and Hung, C.T. Life Sci (19889) 44, 175.

### Definitions

Unless otherwise defined herein, scientific and technical terms used in connection with the present application shall have the meanings that are commonly understood by those of ordinary skill in the art. Further, unless otherwise required by context, singular terms shall include pluralities and plural terms shall include the singular.

As used herein the term "comprising" or "comprises" is used in reference to compositions, methods, and respective component(s) thereof, that are essential to the invention, yet open to the inclusion of unspecified elements, whether essential or not.

As used herein the term "consisting essentially of' refers to those elements required for a given embodiment. The term permits the presence of additional elements that do not materially affect the basic and novel or functional characteristic(s) of that embodiment of the invention.

The term "consisting of' refers to compositions, methods, and respective components thereof as described herein, which are exclusive of any element not recited in that description of the embodiment.

Other than in the operating examples, or where otherwise indicated, all numbers expressing quantities of ingredients or reaction conditions used herein should be understood as modified in all instances by the term "about." The term "about" when used in connection with percentages may mean ±1%. Furthermore, the term "about" can mean within ±1% of a value.

The singular terms "a," "an," and "the" include plural referents unless context clearly indicates otherwise. Similarly, the word "or" is intended to include "and" unless the context clearly indicates otherwise. It is further to be understood that all base sizes or amino acid sizes, and all molecular weight or molecular mass values, given for nucleic acids or polypeptides are approximate, and are provided for description.

Although methods and materials similar or equivalent to those described herein can be used in the practice or testing of this disclosure, suitable methods and materials are described below. The term "comprises" means "includes." The abbreviation, "e.g." is derived from the Latin exempli gratia, and is used herein to indicate a non-limiting example. Thus, the abbreviation "e.g." is synonymous with the term "for example."

The terms "decrease" , "reduced", "reduction" , "decrease" or "inhibit" are all used herein generally to mean a decrease by a statistically significant amount. However, for avoidance of doubt, ""reduced", "reduction" or "decrease" or "inhibit" means a decrease by at least 10% as compared to a reference level, for example a decrease by at least about 20%, or at least about 30%, or at least about 40%, or at least about 50%, or at least about 60%, or at least about 70%, or at least about 80%, or at least about 90% or up to and including a 100% decrease (e.g. absent level as compared to a reference sample), or any decrease between 10-100% as compared to a reference level.

The terms "increased", "increase" or "enhance" or "activate" are all used herein to generally mean an increase by a statically significant amount; for the avoidance of any doubt, the terms "increased", "increase" or "enhance" or "activate" means an increase of at least 10% as compared to a reference level, for example an increase of at least about 20%, or at least about 30%, or at least about 40%, or at least about 50%, or at least about 60%, or at least about 70%, or at least about 80%, or at least about 90% or up to and including a 100% increase or any increase between 10-100% as compared to a reference level, or at least about a 2-fold, or at least about a 3-fold, or at least about a 4-fold, or at least about a 5-fold or at least about a 10-fold increase, or any increase between 2-fold and 10-fold or greater as compared to a reference level.

The term "statistically significant" or "significantly" refers to statistical significance and generally means a two standard deviation (2SD) above or below a reference level. The term refers to statistical evidence that there is a difference. It is defined as the probability of making a decision to reject the null hypothesis when the null hypothesis is actually true. The decision is often made using the p-value.

By "treatment", "prevention" or "amelioration" of a disease or disorder is meant delaying or preventing the onset of such a disease or disorder, reversing, alleviating, ameliorating, inhibiting, slowing down or stopping the progression, aggravation or deterioration the progression or severity of a condition associated with such a disease or disorder. In one embodiment, the symptoms of a disease or disorder are alleviated by at least 5%, at least 10%, at least 20%, at least 30%, at least 40%, or at least 50%.

As used here, the term "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used here, the term "pharmaceutically-acceptable carrier" means a pharmaceutically-acceptable material, composition or vehicle, such as a liquid or solid filler, diluent, excipient, manufacturing aid (e.g., lubricant, talc magnesium, calcium or zinc stearate, or steric acid), or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the patient. Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, methylcellulose, ethyl cellulose, microcrystalline cellulose and cellulose acetate; (4) powdered tragacanth; (5) malt; (6) gelatin; (7) lubricating agents, such as magnesium stearate, sodium lauryl sulfate and talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol (PEG); (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) pH buffered solutions; (21) polyesters, polycarbonates and/or polyanhydrides; (22) bulking agents, such as polypeptides and amino acids (23) serum component, such as serum albumin, HDL and LDL; (22) C₂-C₁₂ alchols, such as ethanol; and (23) other non-toxic compatible substances employed in pharmaceutical formulations. Wetting agents, coloring agents, release agents, coating agents, sweetening agents, flavoring agents, perfuming agents, preservative and antioxidants can also be present in the formulation. The terms such as "excipient", "carrier", "pharmaceutically acceptable carrier" or the like are used interchangeably herein.

As used herein, the term "polymer" is intended to include both oligomeric and polymeric species, i.e., compounds which include two or more monomeric units, which may be a homopolymer or a copolymer. The term "homopolymer" is a polymer incorporating a single species of monomer units. The term "copolymer" is a polymer constructed from two or more chemically distinct species of monomer units in the same polymer chain. A "block copolymer" is a polymer which incorporates two or more segments of two or more distinct species of homopolymers or copolymers.

As used herein, the term "swelling agent" refers to those compounds or substances which affect at least a degree of swelling. Typically, swelling agents is an aqueous solution or organic solvent, however swelling agent can also be a gas. In some emodiments, swelling agent is water or a physiological solution, e.g. phosphate buffer saline, or growth media.

As used herein, the term "linker" means an organic moiety that connects two parts of a compound. Linkers typically comprise a direct bond or an atom such as oxygen or sulfur, a unit such as NH, C(O), C(O)NH, SO, SO₂, SO₂NH or a chain of atoms, such as substituted or unsubstituted C₁-C₆ alkyl, substituted or unsubstituted C₂-C₆ alkenyl, substituted or unsubstituted C₂-C₆ alkynyl, substituted or unsubstituted C₆-C₁₂ aryl, substituted or unsubstituted C₅-C₁₂ heteroaryl, substituted or unsubstituted C₅-C₁₂ heterocyclyl, substituted or unsubstituted C₃-C₁₂ cycloalkyl, where one or more methylenes can be interrupted or terminated by O, S, S(O), SO₂, NH, C(O).

As used herein, a "subject" means a human or animal. Examples of subjects include primates (e.g., humans, and monkeys).Usually the animal is a vertebrate such as a primate, rodent, domestic animal or game animal. Primates include chimpanzees, cynomologous monkeys, spider monkeys, and macaques, e.g., Rhesus. Rodents include mice, rats, woodchucks, ferrets, rabbits and hamsters. Domestic and game animals include cows, horses, pigs, deer, bison, buffalo, feline species, e.g., domestic cat, canine species, e.g., dog, fox, wolf, avian species, e.g., chicken, emu, ostrich, and fish, e.g., trout, catfish and salmon. Patient or subject includes any subset of the foregoing, e.g., all of the above, but excluding one or more groups or species such as humans, primates or rodents. In certain embodiments of the aspects described herein, the subject is a mammal, e.g., a primate, e.g., a human. The terms, "patient" and "subject" are used interchangeably herein. The terms, "patient" and "subject" are used interchangeably herein. A subject can be male or female.

Preferably, the subject is a mammal. The mammal can be a human, non-human primate, mouse, rat, dog, cat, horse, or cow, but are not limited to these examples. Mammals other than humans can be advantageously used as subjects that represent animal models of conditions or disorders associated with decreased spine/excitatory synapse formation and/or numbers. In addition, the methods and compositions described herein can be used to treat domesticated animals and/or pets.

The present disclosure can be further described by any of the following numbered paragraphs:
1. A composition comprising a matrix material and a magnetic material distributed therethrough, wherein the composition comprises pores having mean pore diameter in range of about 10 µm to about 1000 µm, and wherein porosity, pore size, pore connectivity, swelling agent concentration and/or specific volume of the composition undergoes a change from a first value to a second value in response to an electromagnetic signal.
2. The composition of paragraph 1, wherein the composition comprises 0.1%-50% of matrix material, 1-90% of magnetic material.
3. The composition of any of paragraphs 1-2, wherein the composition comprises a swelling agent.
4. The composition of any of paragraph 1-3, wherein the composition comprises 0.1-50% of matrix material, 1-90% of magnetic material and a swelling agent in an amount corresponding to the balance up to 100%.
5. The composition of any of paragraphs 1-4, wherein the composition comprises ∼1% of matrix material, ∼13% magnetic material, and ∼86% of a swelling agent, by weight.
6. The composition of any of paragraphs 1-5, wherein the composition comprises pores having mean pore diameter in range of about 200 µm to about 1000µm.
7. The composition of any of paragraphs 1-6, wherein the composition comprises pores having mean pore diameter of 700µm.
8. The composition of any of paragraphs 1-7, wherein the composition has porosity of 0.1 to 0.99.
9. The composition of any of paragraphs 1-8, wherein the composition has porosity of at least 0.9.
10. The composition of any of paragraphs 1-9, wherein the matrix material is a polymer, copolymer, or blockpolymer gel.
11. The composition of any of paragraphs 1-10, wherein the matrix material is a cross-linked polymer, copolymer, or blockpolymer gel.
12. The composition of paragraph 11, wherein the matrix comprises 0.01 mol % to 15 mol % of a cross-linking agent.
13. The composition of any of paragraphs 1-12, wherein the matrix material comprises a monomer selected from the group consisting of acrylic acid, methyl methacrylate, methyl acrylic acid, ethyl acrylate, vinyl sulfonic acid, styrene, styrene sulfonic acid (e.g., p-styrene sulfonic acid), maleic acid, butenoic acid, vinyl phosphate, vinyl phosphonate, ethylene, propylene, styrene, vinyl methyl ether, vinyl acetate, vinyl alcohol, acrylonitrile, acrylamide, N-(C₁-C₆ alkyl) acrylamide (such as N-isopropylacrylamide, N-t-butylacrylamide), and combinations thereof.
14. The composition of any of paragraphs 1-13, wherein the matrix material comprises a polymer selected from the group consisting of glycosaminoglycan, silk, fibrin, MATRIGEL®, poly-ethyleneglycol (PEG), polyhydroxy ethyl methacrylate, polyvinyl alcohol, polyacrylamide, poly (N-vinyl pyrolidone), poly(lactic acid), poly glycolic acid (PGA), poly lactic-co-glycolic acid (PLGA), poly e-carpolactone (PCL), polyethylene oxide, poly propylene fumarate (PPF), poly acrylic acid (PAA), polyhydroxybutyric acid, hydrolysed polyacrylonitrile, polymethacrylic acid, polyethylene amine, esters of alginic acid; pectinic acid; and alginate, fully or partillay oxidized alginate, hyaluronic acid, carboxy methyl cellulose, heparin, heparin sulfate, chitosan, carboxymethyl chitosan, chitin, pullulan, gellan, xanthan, collagen, gelatin, carboxymethyl starch, carboxymethyl dextran, chondroitin sulfate, cationic guar, cationic starch, and combinations thereof.
15. The composition of any of paragraphs 1-14, wherein the magnetic material is ferromagnetic, ferromagnetic, diamagnetic, paramagnetic, or superparamagnetic.
16. The composition of any of paragraphs 1-15, wherein the magnetic material is selected from group rare earth metals, transient metals, noble metals, and oxides, compositions, combinations, solid dispersions, and alloys thereof.
17. The composition of any of paragraphs 1-16, wherein the magnetic material is selected from the group consisting of maghemite (Fe₂O₃), magnetite (Fe₃O₄), strontium ferrite, samarium-cobalt, neodymium-iron-boron (NIB), lodestone, pyrrhotite, BaFe₁₂O₁₉, Alnico magnet alloy, transfer salts of decamethylmetallocenes with 7,7,8,8-tetracyano-p-quinodimethane (TCNQ) or tetracyanoethenide (TCNE), hexylammonium trichlorocuprate(II) (CuCl₃(C₆H₁₁NH₃), Fe based amorphous magnetic powders, and combinations thereof.
18. The composition of any of paragraph 1-17, wherein the magnetic material is a magnetic particle having a size from about 1 nm to 1000 µm.
19. The composition of any of paragraphs 1-18, wherein the magnetic material is a magnetic nano-particle.
20. The composition of any of paragraphs 1-19 wherein the composition comprises a bioactive agent.
21. The composition of paragraph 20, wherein the bioactive agent is covalently linked to the matrix.
22. The composition of paragraphs 20 or 21, wherein the bioactive agent is a therapeutic agent.
23. The composition of any of paragraphs 1-22, wherein the composition comprises a cell.
24. The composition of paragraph 23, wherein the cell is a human cell, a mouse cell or a rat cell.
25. The composition of any of paragraphs 1-24, wherein porosity, pore size, pore connectivity, swelling agent concentration, and/or specific volume changes by at least 10%, 15%, 20%, 25% 30%, 40%, 50%, 60%, 70%, 80%, 90%, 95%, 96 %, 97%, 98%, 99% or more relative to the original value on application of a magnetic field.
26. The composition of any of paragraphs 1-25, wherein porosity, pore size, pore connectivity, swelling agent concentration, and/or specific volume decreases on application of a magnetic field.
27. The composition of any of paragraphs 1-26, wherein the electromagnetic signal has a defined magnetic field strength
28. The composition of paragraph 27, wherein the magnetic field strength is from about 0.001 Tesla to about 1 Tesla.
29. The composition of any of paragraphs 1-28, wherein the matrix material comprises a cell binding molecule.
30. The composition of paragraph 29, wherein the cell binding molecule is selected from the group consisting of antibodies, intrabodies, antigens, lectins, proteins, peptides, nucleic acids, receptor molecules, ligands for receptors, and any combinations thereof.
31. The composition of any of paragraphs 29 or 30, wherein the cell binding molecule is an integrin-binding peptide.
32. The composition of any of paragraphs 29-31, wherein the cell binding molecule is a peptide comprising the amino acid sequence Arg-Gly-Asp (RGD).
33. The composition of any of paragraphs 29-33, wherein the cell binding molecule is a peptide comprising the amino acid sequence (Gly)₄-Arg-Gly-Asp-Ala-Ser-Ser-Lys-Tyr (SEQ ID NO: 1).
34. The composition of any of paragraphs 1-33, wherein the magnetic material is distributed homogeneously within the matrix material.
35. The composition of any of paragraphs 1-33, wherein the magnetic material is distributed heterogeneously within the matrix material.
36. The composition of any of paragraphs 1-35, wherein the the composition comprises a compound selected from the group consisting of small organic or inorganic molecules; saccharines; oligosaccharides; polysaccharides; peptides; proteins; peptide analogs and derivatives; peptidomimetics; nucleic acids; nucleic acid analogs and derivatives; an extract made from biological materials such as bacteria, plants, fungi, or animal cells; animal tissues; naturally occurring or synthetic compositions; and any combinations thereof.
37. The composition of paragraph 36, wherein the compound is covalently linked to the matrix.
38. The composition of paragraph 38, wherein the compound has biological activity.
39. A method for preparing the composition of any of paragraphs 1-38, method comprising: (a) preparing a composition comprising a magnetic material; (b) freezing the composition of step (a), wherein freezing temperature is from about -4°C to about -180°C; (c) lyophilizing the frozen composition; (d) swelling the lyophilized composition.
40. The method of paragraph 39, wherein freezing temperature is from about -10°C to about -50°C.
41. The method of any of paragraphs 39 or 40, wherein freezing temperature is about - 20°C.
42. A method for controlling the release of a bioactive agent, the method comprising: (a) providing to a subject a composition of any of paragraphs 1-38, wherein the composition comprises the bioactive agent; and (b) inducing a change in porosity, pore size, pore connectivity, swelling agent concentration, and/or specific volume of the composition via a magnetic field to control the release of the bioactive agent.

To the extent not already indicated, it will be understood by those of ordinary skill in the art that any one of the various embodiments herein described and illustrated can be further modified to incorporate features shown in any of the other embodiments disclosed herein.

The following examples illustrate some embodiments and aspects of the invention.

### EXAMPLES

### Example 1: Unmodified macroporous ferrogels

**Preparation of macroporous ferrogels.** FeCl₂.4H₂O, FeCl₃.6H₂O and NH₄OH were used to prepare magnetite particles by the chemical co-precipitation method as described in Betancourt-Galindo et al, J. Magn. Magn. Mater. (2005), 294: 33. The average diameter of the magnetite particles obtained was ∼300nm. The magnetite particles were further coated with polymers such as poly(acrylamide-co-acrylichydrazide) with molecular weight ∼1500Dalton. The coated magnetite particles were mixed with alginate solution. The resulted alginate solution was immediately crosslinked with adipic acid dihydrazide to form a magnetic alginate hydrogel according to the method described in F. Bueche, F. and Halpin, J.C., Journal of Applied Physics (1964), 35:36. The weight ratio of magenetic nanoparticle, polymer, and water in the final solution was 13%, 1%, and 86%, respectively.

Magentite particle comprising gels were frozen at temperature raging from -20°C to -180°C (e.g., at -20°C, -80°C, and -180°C), and then lyophilized. The freeze-lyophilize process introduced macropores with diameters ranging from 700µm to 20µm into the gels. Table 1 lists average pore diameter formed at various temperatures.

**Table 1. Average pore diameters of unmodified macroporous ferrogels.**

| **Freeze temp. (°C)** | **Avg. pore diameter** |
|---|---|
| -20 | 700µm |
| -80 | 200µm |
| -180 | 20µm |

The macroporous gels were re-swollen in de-ionized water. The weight concentration of polymer, magnetite particles and water was ∼1%, ∼13%, and ∼86% in the final macroporous gel. Upon application of a magnetic filed, the gels underwent a volume change of about 75% (Fig. 2c) in comparison to a common ferrogel having a similar composition but average pore diameter of less than 100 nm .

### Example 2: RGD modified macroporous ferrogels.

**Materials.** Sodium alginate with high guluronate content (Protanal LF 20/60) was purchased from Pronova Biopolymers Inc. (Portsmouth, NH) and used without further purification. Phosphate Buffered Saline (PBS) was purchased from Invitrogen (Carlsbad, CA). Adipic acid dihydrazide (AAD), 1-ethyl-3-(dimethylaminopropyl) carbodiimide (EDC), 2-(N-morpholino)ethanesulfonic acid hydrate (MES), 1-hydroxybenzotriazole (HOBt), Iron(III) chloride hexahydrate, oleic acid, 1-octadecene, cetyltrimethylammonium bromide (CTAB) were purchased from Sigma-Aldrich (St. Louis, MO). Sodium oleate was purchased from TCI (Portland, OR). Pluronic F127 was purchased from BASF (Florham Park, NJ). The integrin-binding peptide (Gly)₄-Arg-Gly-Asp-Ala-Ser-Ser-Lys-Tyr was purchased from Commonwealth Biotech (Richmond, VA).

**Macroporousferrogel fabrication.** The Fe₃O₄ nanoparticles were fabricated according to method described in Park, et al. (Nat. Mater. 2004, 3, 891-895) and the surfactant, Pluronic F127, was coated on the nanoparticles following the procedure described in Qin, et al. (Adv. Mater. 2007, 19, 1874-1878). Alginates were coupled with RGD following aqueous carbodiimide chemistry as described in Drury, J. L. & Mooney, D. J.(2003) Hydrogels for tissue engineering: scaffold design variables and applications Biomaterials, 2003, 24, 4337-4351. To prepare macroporousferrogels, RGD-modified alginate in MES buffer (0.1 M MES and 0.5 M NaCl, pH 6.0) was sequentially mixed with an aqueous solution of Fe₃O₄ nanoparticles, HOBt, EDC, and AAD. The concentration of alginate was 1 wt %, Fe₃O₄ was 13 wt %, and AAD was 5mM in the resulting solution. A baseline RGD density was set to 7.43 µmol RGD per gram of alginate according to Rowley, J. A., Madlambayan, G., & Mooney, D. J.(Biomaterials, 1999, 20, 45-53). The mixture was immediately cast between two glass plates separated by 4∼11 mm spacers. After 2 h, the ferrogel was cut into various shapes and placed in a large volume of distilled water, for a minimum of 24 hr, to remove any residual reagents. The gels were then frozen at -20°C, - 80°C, or -180°C and lyophilized to generate macroporousferrogels with variable pore characteristics.

**SEM sample preparation.** The SEM samples in Fig 1b were prepared by sectioning the as-prepared macroporousferrogels before hydration in water. To prepare the SEM samples in Fig 2c, hydrated macroporousferrogels were frozen in the undeformed and deformed states in liquid nitrogen, and then lyophilized and sectioned for observation.

**Pore size and connectivity evaluation.** The average sizes of the pores in macroporous gels were calculated by averaging the diameters of the pores in the gels observed by SEM. In order to assess the pore connectivity, the gels were soaked in a solution containing 1.0x10⁸/ml of FluoSpheres red fluorescent microspheres (Molecular Probes) with 1 um diameter, and subject to 120 cycles (on/off) of the external magnetic field. The gels were then dehydrated and examined using micro-computed tomography (CT) to determine the distribution of the fluorescent beads within the gels. Micro-CT images visualized were obtained from the mid plane (2.5 mm above from the bottom of gels) of the cylindrical shaped-ferrogel scaffolds (5 mm in height). Fluorescents beads were imaged at high resolution (HMXST225, X-Tek; Nikon Metrology NV, Leuven, Belgium) with the scanner set to a voltage of 100 kV and a current of 100 A. Next, a series of 2D images of each sample were taken with an X-Tek x-ray machine while the sample was rotated 360 degree. The series of 2D images were converted to 3D images using CT Pro software. Image rendering and subsequent image processing including movie production were performed with Volume Graphics (VG) studio MAX (Heidelberg, Germany). The pore connectivity was further evaluated using a water wicking technique in which the interconnected porosity was calculated as the interconnected void volume/total volume. To determine total volume, gels were soaked in water for 1 hour and weighed. A Kimwipe was then used to wick away water within interconnected pores and the gels were weighed once again. The interconnected void volume was calculated as the volume of water wicked from the gels.

**Mechanical testing.** The nanoporousferrogel and macroporousferrogel were cut into cylinders (15mm diameter, 8mm height). The cylinders were subject to compression tests using an Instron 3342 from Instron (Norwood, MA) with a strain rate of 20% per minute. Engineering stresses and strains were recorded. The ferrogel cylinders were kept hydrated throughout the tests.

**Controlled release of mitroxantrone.** The macroporousferrogels in dry state were cut into disks with a volume ∼2mL. Each disk was allowed to absorb 1 mL of an aqueous solution of mitoxantrone with a concentration of 300mg/mL. The resulting gels were stored at room temperature for ∼6 hours. The macroporousferrogels were then placed in a phosphate buffered saline (PBS) solution. Every 30 min, one group of the macroporousferrogels was subjected to 120 cycles (on/off) of the magnetic field over 2 min, while no magnetic field was applied to the other gels. The medium surrounding each disk was collected and replaced with fresh buffer every 30 min and after each period of magnetic stimulation. Mitoxantrone concentration was recorded by measuring the absorbance of mitroxantrone in PBS solution at a wavelength of 327.5nm.

**Controlled release of plasmid DNA.** Plasmid DNA (Aldevron) was combined with linear polyethyleneimine (PEI) (Fermentas) to form a polyelectrolyte complex as described in Kong, H. J., Hsiong, S., & Mooney, D. J.(Nano Lett. 2007, 7, 161-166. The ratio of the number of amine groups in PEI to the number of phosphate groups (N/P ratio) in DNA was kept constant at 7. Macroporousferrogels with a volume ∼1mL were allowed to absorb 0.5 mL of an aqueous solution of plasmid DNA-PEI complex with a DNA concentration of 600ug/mL. Thereafter, ferrogel disks were used for the release study following a similar procedure as described in controlled release of mitroxantrone, except the magnetic field was applied every 2 hours instead of every 30 minutes. The medium surrounding each disk was collected and replaced with fresh buffer every 2 hours and after each period of magnetic stimulation. The concentration of plasmid DNA released into the medium was measured using Quant-iT™ PicoGreen® dsDNA Assay Kit (Invitrogen).

**Controlled cell release *in vitro.*** Human dermal fibroblasts (1.5x10⁶) (Lonza) were seeded in each ferrogel gel disk, and the gel disks were incubated at 37°C for 4 hours to allow cell adhesion. Thereafter, the ferrogel disks were placed in FBS-supplemented DMEM and the cells were released following a similar procedure as described in controlled release of mitroxantrone, except the magnetic field was applied every 2 hours instead of every 30 minutes. The medium surrounding each disk was collected and replaced with fresh DMEM every 2 hours and after each period of magnetic stimulation. The numbers of cells released were counted with a Z2™ Coulter Counter (Beckman Coulter). The cells released by magnetic stimulation were also incubated in a Petri dish with DMEM at 37°C to monitor adhesion and proliferation.

**Controlled cell release *in vivo.*** Mouse mesenchymal stem cells (1.5x10⁶) (ATCC) stained with DiOC18 (Invitrogen) according to manufacturer's protocol were seeded in each ferrogel gel disk, and the gel disks were incubated at 37°C for 4 hours to allow cell adhesion. The gel disks were implanted subcutaneously into the back region of female nude mice (NU/J, Jackson Laboratory) under anesthesia, and the incisions were closed by 5-0 Ethilon sutures (Johnson & Johnson). In order to create a space for placement of gels and cell release, liquid pockets were generated around the ferrogels by subcutaneous PBS injection prior to gel placement. One hour after the implantation, the ferrogels were subject to 120 cycles (on/off) of the external magnetic field by approaching and retracting a magnet against the skin of the mouse. Fluorescence images of mice were obtained on a Xenogen IVIS Spectrum system (Caliper Life Sciences, Hopkinton, MA). Animal work was performed in compliance with NIH and institutional guidelines.

### Results

Macroporousferrogels were first developed, in order to allow for bioactive agents ranging from molecules to cells to be encapsulated and released on demand. Macroporous gels with a hierarchal structure (**Fig. 1**) of various sizes (mm-cm or larger) and shapes (**Fig. 1a**) can readily be fabricated to accommodate the specific application. Pores with controlled sizes and connectivity were introduced into the ferrogels by freezing gels at different temperatures prior to lyophilization (Thornton, et al., Transplantation, 2004, 77, 1798-1803. Corresponding to freezing temperatures of -20°C, -80°Cand -180°C, the average diameters of the pores were 700µm, 300µm and 20µm. Highly interconnected pores were observed in gels prepared at -20°C, and -80°C, while poorly interconnected pores were observed in gels prepared at -180°C (data not shown). This finding agrees with the earlier reports on alginate macroporous gels (Thornton, et al., Transplantation, 2004, 77, 1798-1803 and Thornton, et al., J. Urol. 2004, 172, 763-768). Highly monodisperse iron oxide nanoparticles with a diameter ∼10 nm were embedded in the gel (Fig. 1c) at predetermined concentrations (left 13 wt %, and right 4 wt %) (Thornton, et al., J. Urol. 2004, 172, 763-768). The surfaces of the nanoparticles were pre-coated with Pluronic F127 (Qin, et al., Adv. Mater. 2007, 19, 1874-1878) to minimize agglomeration, and this resulted in a relatively homogeneous distribution of nanoparticles in the gels (**Fig. 1d**). Alginate gels were covalently cross-linked with adipic acid dihydrazide (AAD) (**Fig.1d**) (Thornton, et al., Transplantation, 2004, 77, 1798-1803), as this allowed gels to maintain the macroporous structure following lyophilization and subsequent rehydration. Peptides containing the arginine-glycine-aspartic acid (RGD) amino acid sequence were covalently coupled to the polymer prior to gel formation (**Fig. 1d**) (Drury, J. L. & Mooney, D. J. Biomaterials, 2003, 24, 4337-4351). The RGD coupling confers a specific mechanism for integrin-mediated cell adhesion to the otherwise nonadhesive polymer, and the RGD density can be manipulated to provide control over cell adhesion.

The influence of the macropores on the gel mechanical properties were next evaluated, as the stiffness of the gels will dictate the extent of deformation that will result from a specific magnetic field. It is well established that by creating pores in a gel, one can greatly reduce its elastic rigidity (Gibson, L. J. & Ashby, M. F. Cellular solids, 1997, Cambridge University Press, Cambridge). A ferrogel fabricated with 13 wt % Fe₃O₄ and 1 wt % alginate crosslinked by 5mM AAD gives an initial Young's modulus (i.e. the slope of the initial part of the stress vs. strain curve in **Fig. 2a**) of ∼30KPa in a compression test. Introducing connected pores of ∼700 µm diameter into the ferrogel led to a dramatic reduction in the initial modulus, to ∼2.5KPa (**Fig. 2a**).These macroporous gels can also be reversibly deformed to a compressive strain of over 80% before fracture, in contrast to the more brittle nature of the standard nanoporous alginate gels (**Fig. 2a**). In addition, it has been observed that the initial Young'smoduli of the macroporous gels decrease with the rise of the freezing temperature used in preparation of the gels (**Fig. 6**). Macroporousferrogels fabricated with 13 wt % Fe₃O₄ and 1 wt % alginate crosslinked by 5mM AAD, and freezing at -20°C were used for the remaining experiments. The macroporousferrogels with interconnected pores, larger pore size (**Fig. 1b**), higher concentration of iron oxide particles (**Fig. 1c**) and lower modulus (**Fig. 6**) were chosen because: a) Drug and cell transport will presumably be more efficient in scaffolds with larger pore sizes and better connectivity (Thornton, A. J., Alsberg, E., Albertelli, M., & Mooney, D. J. Transplantation,. 2004, 77, 1798-1803); b) a higher iron-oxide-particle density gives a higher body force under the same magnetic field; and c) a gel with lower modulus tends to deform more when subject to the same body forces. Magnetic-field-induced large deformation of the gel leads to more pronounced effects on drug and cell release.

The deformation of nanoporous and macroporousferrogels under the influence of a moderate magnetic field was next examined. Subject to a non-uniform magnetic field, a ferrogel experiences a body force proportional to the gradient of the applied field, and this will result in a shape change (Zrinyi, M., Barsi, L., & Buki, A. Polym. Gels Netw. 1997, 5, 415-427 and Zrinyi, M., Barsi, L., & Buki, A. J. Chem. Phys. 1996, 104, 8750-8756). A cylinder of a typical nanoporousferrogel reduced its height by ∼5% when subjected to a vertical magnetic-field gradient of ∼38 A/m², using a magnetic bar placed at the bottom of the gel (**Fig. 2b**). In comparison, a cylinder of the macroporousferrogel gave a much larger deformation, ∼70%, under the same magnetic field (**Fig. 2c**), due to its lower modulus (**Fig. 2a**). In addition, the cylinder of the macroporous gel also reduced its volume by ∼70% (**Fig. 2c**), as contrasted to a minimal volume change of the nanoporous gels (**Fig. 2b**). The large volume change of the macroporous gels was caused by collapse of the pores in these gels, as demonstrated by SEM images of freeze-dried macroporousferrogels in the deformed and undeformed states (**Fig. 2d**). The collapsing pores can force water contained in the connected pores to flow out of the gel. Once the magnetic field is off, the elastically deformed gel quickly returned to its original, undeformed configuration in less than one second, as surrounding water was reabsorbed into the gel (data not shown).

Without wishing to be bound by theory, the magnetic-field-induced gel deformation and resulting water convection and associated shear forcescould accelerate the release of drugs encapsulated in ferrogels, or cells adherent to the pore surfaces of the macroporousferrogels. To demonstrate this, the inventors first chose mitoxantrone (MW 444 g/mol), an antineoplastic agent, as a model drug. Mitoxantrone forms an ionic complex with the carboxylate groups on alginate, which retards its release (Bouhadir, K. H., Alsberg, E., & Mooney, D. J. Biomaterials, 2001, 22, 2625-2633. Macroporousferrogels each containing 300mg mitoxantrone were placed in a phosphate buffered saline (PBS) solution. Every 30 min, one group of the macroporousferrogels was subjected to 120 cycles (on/off) of the magnetic field over 2 min by manually approaching and retracting a magnet against the gels, while no magnetic field was applied to the other gels. The rapid recovery time of the gels allowed them to recover their initial dimensions in each cycle. The macroporous gel deformed reversibly and promptly in response to the cycling magnetic field, and the applied magnetic stimulation greatly accelerated the release of mitoxantrone, as sheen grossly by the increasing blue color in the PBS (data not shown). Gel deformation and water convection promotes dissociation of drug from the polymer, and enhances transport of unbound drug out of the gel. The cumulative release profile showed a stepwise increment with magnetic stimulation, and the total amount of mitoxantrone released from the stimulated gel was ∼7 times more than that of the control case after 3 hours (**Fig. 3a**). This study was repeated with a higher molecular-weight biological agent, plasmid DNA (MW ∼10⁶ g/mol) condensed with polyethyleneimine (PEI, MW ∼22,000 g/mol), to determine if this approach would have utility with a wide range of potential drugs. Again, significant stepwise increases in release was noted with application of the magnetic field, as compared to control gels (**Fig. 3b**).

Next, controlled cell release from the macroporous gels *in vitro* was examined (**Fig. 4a**). The RGD on alginate in macroporousferrogels enables cells to adhere on the porous surfaces, and the adhesion strength increases with RGD density (Xiao, Y. & Truskey, G. A. Biophys. J. 1996, 71, 2869-2884. A baseline RGD density was set to 7.43 µmol RGD per gram of alginate (Rowley, J. A., Madlambayan, G., & Mooney, D. J. Biomaterials, 1999, 20, 45-53, and 50% and 10% of the baseline RGD density were also used to examine the role of gel adhesiveness on cell release. Human dermal fibroblasts (1.5x10⁶) were seeded in each gel, and gels were incubated at 37°C for 4 hours to allow cell adhesion. Thereafter, the macroporousferrogels were subject to 120 cycles (on/off) of the magnetic field for ∼2 min, at 2-hour intervals. The cumulative release of cells was quantified as a function of time (Fig. 4b). Magnetic stimulation resulted in burst release of cells, and the release profile varied for gels with different RGD densities. After 6 hours (3 rounds of magnetic stimulation), around a third of the cells were released from gels with the baseline RGD density, while over half of the cells were released from ferrogels with 50% less RGD. The gels with only 10% of the baseline RGD content delivered over 90% of their cells after 4 hours (2 rounds of stimulation). These results are consistent with the lower RGD densities providing weaker cell adhesion, resulting in more cell detachment and release under the magnetic stimulation (Dainiak, et al., Proc. Natl. Acad. Sci. U. S. A. 2006, 103, 849-854). The released cells were collected and probed for viability. More than 95% of the released cells were viable (data not shown). The released cells were also incubated in a Petri dish at 37°C. The released cells spread normally, and became confluent after 2 days (**Fig. 4c**), indicating that released cells remained viable and functional.

The inventors also examined the capability of macroporousferrogels in controlled delivery of cells *in vivo.* Mouse mesenchymal stem cells (1.5x10⁶) stained with DiOC18, a membrane dye with near infrared emission maximum, were seeded in macroporousferrogels with 50% baseline RGD content. The gels were implanted into the subcutaneous space of mice. One hour after the implantation, the gels were subjected to 120 cycles (on/off) of the external magnetic field by approaching and retracting a magnet against the mouse skin over the gel. As a control, implanted ferrogels were subject to no magnetic stimulation, and *in vivo* fluorescence images of both conditions were obtained before and after magnetic stimulation (**Fig. 5**). The control mouse (left in **Figs. 5a and 5b**) showed almost no change in fluorescence, indicating few cells were released from the ferrogel. In contrast, application of the external magnetic field led to a significant increase in fluorescence around the ferrogel, indicating a burst release of stem cells.

### Discussion

The results of this study demonstrate an on-demand and reversible approach for delivering various biological agents from a macroporousferrogel of the invention, in response to external magnetic fields. A new delivery mechanism based on deformation of macroporousferrogels under magnetic stimulation, and subsequent water flow through connected pores was demonstrated. By creating large pores in a ferrogel, one can greatly reduce the rigidity of the gel and increase its hydraulic conductivity (Gibson, L. J. & Ashby, M. F. Cellular solids, 1997, Cambridge University Press, Cambridge). As a result, the macroporousferrogels demonstrate very large deformation, volumetric change, and water convection under applied magnetic fields, in contrast to the minimal values of conventional nanoporousferrogels. Furthermore, it is generally difficult for nanoporousferrogels to release drugs with high molecular weight, such as proteins and plasmid DNA, due to the limited mobility of these drugs in the nanopores. See for example, Lu, et al., Langmuir 2005, 21, 2042-2050; Hu, et al., Macromolecules, 2007, 40, 6786-6788; Hu, et al., Adv. Mater. 2008, 20, 2690; Liu, et al., Langmuir 2006, 22, 5974-5978; Hu, et al., J. Control. Release, 2007, 121, 181-189; and Resendiz-Hernandez, P. J., Rodriguez-Fernandez, O. S., & Garcia-Cerda, L. A. J. Magn. Magn. Mater. 2008, 320, E373-E376. The connected macropores in the new ferrogel enable the rapid transport of various drugs ranging from small molecules such as mitoxantrone to very large molecules such as the protein SDF-1α and plasmid DNA out of the gel, upon magnetic stimulation. This is consistent with previous observations that mechanical compression of macroporous or nanoprous gels can enhance the release of biological agents. See for example, Dainiak, et al., Proc. Natl. Acad. Sci. U. S. A. 2006, 103, 849-854 and Lee, et al., Nature, 2000, 408, 998-1000. Here, the inventors have discovered that reversible deformation of macroporousferrogels allows a noval mechanism for on-demand drug and cell release under the control of external magnetic fields.

These scaffolds also act as active depots of various cells whose release can be controlled over time. The polymers used to form conventional ferrogels typically do not support cell adhesion (Hu, et al., Macromolecules, 2007, 40, 6786-6788 and Resendiz-Hernandez, P. J., Rodriguez-Fernandez, O. S., & Garcia-Cerda, L. A. J. Magn. Magn. Mater. 2008, 320, E373-E376). The ferrogels of the invention overcome this issue. The polymer used to form the ferrogels is covalently coupled with cell-binding peptides, as the peptide density can be manipulated to provide control over cell adhesion. On-demand release of human dermal fibroblasts and mouse mesenchymal stem cells were demonstrated *in vitro* and *in vivo* under the control of external magnetic fields. This is the first demonstration, to the best of our knowledge, of the use of macroporousferrogels for controlled cell delivery. More broadly, this provides the first demonstration of on-demand release of cells from porous scaffolds, and this is of wide utility for tissue regeneration and cell therapies. The peptide-modified macroporous ferrogel maintains the adhesion and viability of the resident cells, and subjecting the gels to external magnetic stimulation allows one to release a prescribed number of the resident cells on demand over various time-frames. Multiple parameters in this delivery system are tunable, including peptide density, the strength of applied magnetic field, number of magnetic cycles, and frequency of magnetic stimulation, to control the release of various cell types.

Accordingly, the invention provides an active scaffold in the form of a macroporous ferrogel, which is responsive to magnetic fields. Ferrogels consisting of magnetic particles embedded in polymer gels have been intensively investigated, due to the broad application and clinical acceptance of magnetic particles and magnetic fields. See, for example, Weissleder, et al., Adv. Drug Deliv. Rev.i 1995, 16, 321-334; Brigger, I., Dubernet, C., & Couvreur, P. Adv. Drug Deliv. Rev. 2002, 54, 631-651; Lu, A. H., Salabas, E. L., & Schuth, F. Angew. Chem.-Int. Edit. 2007, 46, 1222-1244; Gupta, A. K. & Gupta, M. Biomaterials, 2005, 26, 3995-4021; Pankhurst, et al., J. Phys. D-Appl. Phys. 2003, 36, R167-R181; and Mornet, et al., J. Mater. Chem. 2003, 14, 2161-2175. Recent studies have shown controlled release of a number of drugs from ferrogels subject to magnetic fields. See for example, Lu, et al., Langmuir 2005, 21, 2042-2050; Hu, et al., Macromolecules, 2007, 40, 6786-6788; Hu, et al., Adv. Mater. 2008, 20, 2690; Liu, et al., Langmuir 2006, 22, 5974-5978; Hu, et al., J. Control. Release, 2007, 121, 181-189; and Resendiz-Hernandez, P. J., Rodriguez-Fernandez, O. S., & Garcia-Cerda, L. A. J. Magn. Magn. Mater. 2008, 320, E373-E376. Ferrogels have also been made biodegradable (Chatterjee, J., Haik, Y., & Chen, C. J. Colloid Polym. Sci. 2003, 281, 892-896) and injectable (Qin, et al., Adv. Mater. 2009, 21, 1354-1357). However, typical ferrogels for drug delivery demonstrate very limited deformation and volumetric change, and the pore sizes in most ferrogels are in the nanometer range (Hu, et al., J. Control. Release, 2007, 121, 181-189), which limits transport of large molecules and cells through the gel. Cell delivery from ferrogels is particularly difficult, and the polymers typically used to fabricate ferrogels do not support cell adhesion (Lu, et al., Langmuir 2005, 21, 2042-2050 and Resendiz-Hernandez, P. J., Rodriguez-Fernandez, O. S., & Garcia-Cerda, L. A. J. Magn. Magn. Mater. 2008, 320, E373-E376). The invention solves this problem and provides magnetic-sensitive scaffolds capable of on-demand drug and cell delivery. The polymer gels were fabricated with three-dimensionally connected macropores and coupled with magnetic nanoparticles and cell-binding peptides. Alginate, a naturally occurring polysaccharide, which comprises α-L-guluronic and β-D-mannuronic acid sugar residues, was used to fabricate the scaffolds, as it had been used extensively in biomedical applications. Peptides containing the arginine-glycine-aspartic acid (RGD) amino acid sequence, a ubiquitous cell-binding domain found in many extracellular matrix molecules, were covalently coupled to the alginate. Iron oxide particles with diameters ∼10 nm were embedded in the alginate, which results in a superparamagnetic gel (Qin, et al., Adv. Mater. 2009, 21, 1354-1357). Macroporous structures with interconnected pores in the micrometer range were generated from the gels. Under applied magnetic fields, the macroporousferrogel can gave large and prompt deformation, causing water flow through the interconnected pores. The deformation and water convection can trigger and enhance release of biological agents. Various drugs and cells were encapsulated in the scaffolds, and their ability to be released under the control of external magnetic fields was probed *in vitro* and *in vivo.*

### Reference

1. Langer, R. & Vacanti, J. P.(1993) Tissue Engineering Science260, 920-926.
2. Griffith, L. G. & Naughton, G.(2002) Tissue engineering - Current challenges and expanding opportunities Science295, 1009-1016.
3. Hutmacher, D. W.(2000) Scaffolds in tissue engineering bone and cartilage Biomaterials21, 2529-2543.
4. Karageorgiou, V. & Kaplan, D.(2005) Porosity of 3D biomaterial scaffolds and osteogenesis Biomaterials26, 5474-5491.
5. Hollister, S. J.(2005) Porous scaffold design for tissue engineering Nat. Mater.4, 518-524.
6. Mroz, T., Yamashita, T., & Lieberman, 1.(2008) The on- and off-label use of rhBMP-2 (INFUSE) in Medicare and non-Medicare patients. Spine J. 8, 41S-42S.
7. Mikos, A. G., Thorsen, A. J., Czerwonka, L. A., Bao, Y., Langer, R., Winslow, D. N., & Vacanti, J. P.(1994) Preparation and Characterization of Poly(L-Lactic Acid) Foams Polymer35, 1068-1077.
8. Mooney, D. J., Baldwin, D. F., Suh, N. P., Vacanti, L. P., & Langer, R.(1996) Novel approach to fabricate porous sponges of poly(D,L-lactic-co-glycolic acid) without the use of organic solvents Biomaterials17, 1417-1422.
9. Shastri, V. P., Martin, I., & Langer, R.(2000) Macroporous polymer foams by hydrocarbon templating Proc. Natl. Acad. Sci. U. S. A.97, 1970-1975.
10. Yang, S. F., Leong, K. F., Du, Z. H., & Chua, C. K.(2001) The design of scaffolds for use in tissue engineering. Part 1. Traditional factors Tissue Eng.7, 679-689.
11. Hofmann, M., Wollert, K. C., Meyer, G. P., Menke, A., Arseniev, L., Hertenstein, B., Ganser, A., Knapp, W. H., & Drexler, H.(2005) Monitoring of bone marrow cell homing into the infarcted human myocardium Circulation111, 2198-2202.
12. Skuk, D., Caron, N. J., Goulet, M., Roy, B., & Tremblay, J. P.(2003) Resetting the problem of cell death following muscle-derived cell transplantation: Detection, dynamics and mechanisms J. Neuropathol. Exp. Neurol.62, 951-967.
13. Evans, S. M., Mummery, C., & Doevendans, P. A.(2007) Progenitor cells for cardiac repair Semin. Cell Dev. Biol.18, 153-160.
14. Thuret, S., Moon, L. D. F., & Gage, F. H.(2006) Therapeutic interventions after spinal cord injury Nat. Rev. Neurosci.7, 628-643.
15. Auger, F. A., Berthod, F., Moulin, W., Pouliot, R., & Germain, L.(2004) Tissue-engineered skin substitutes: from in vitro constructs to in vivo applications Biotechnol. Appl. Biochem.39, 263-275.
16. Nerem, R. M.(2007) Cell-based therapies: From basic biology to replacement, repair, and regeneration Biomaterials28, 5074-5077.
17. Mooney, D. J. & Vandenburgh, H.(2008) Cell delivery mechanisms for tissue repair Cell Stem Cell2, 205-213.
18. Langer, R.(1990) New Methods of Drug Delivery Science249, 1527-1533.
19. Hoffman, A. S.(1995) Intelligent Polymers in Medicine and Biotechnology Artificial Organs19, 458-467.
20. Hsieh, D. S. T., Langer, R., & Folkman, J.(1981) Magnetic Modulation of Release of Macromolecules from Polymers Proceedings of the National Academy of Sciences of the United States of America-Biological Sciences78, 1863-1867.
21. Peppas, N. A., Hilt, J. Z., Khademhosseini, A., & Langer, R.(2006) Hydrogels in biology and medicine: From molecular principles to bionanotechnology Adv. Mater.18, 1345-1360.
22. Galaev, I. Y. & Mattiasson, B.(1999) 'Smart' polymers and what they could do in biotechnology and medicine Trends Biotechnol. 17, 335-340.
23. Miyata, T., Asami, N., & Uragami, T.(1999) A reversibly antigen-responsive hydrogel Nature399, 766-769.
24. Wood, K. C., Zacharia, N. S., Schmidt, D. J., Wrightman, S. N., Andaya, B. J., & Hammond, P. T.(2008) Electroactive controlled release thin films Proc. Natl. Acad. Sci. U. S. A.105, 2280-2285.
25. Mitragotri, S. & Lahann, J.(2009) Physical approaches to biomaterial design Nat. Mater.8, 15-23.
26. Weissleder, R., Bogdanov, A., Neuwelt, E. A., & Papisov, M.(1995) Long-Circulating Iron-Oxides for Mr-Imaging Adv. Drug Deliv. Rev.16, 321-334.
27. Brigger, I., Dubernet, C., & Couvreur, P.(2002) Nanoparticles in cancer therapy and diagnosis Adv. Drug Deliv. Rev.54, 631-651.
28. Lu, A. H., Salabas, E. L., & Schuth, F.(2007) Magnetic nanoparticles: Synthesis, protection, functionalization, and application Angew. Chem.-Int. Edit.46, 1222-1244.
29. Gupta, A. K. & Gupta, M.(2005) Synthesis and surface engineering of iron oxide nanoparticles for biomedical applications Biomaterials26, 3995-4021.
30. Pankhurst, Q. A., Connolly, J., Jones, S. K., & Dobson, J.(2003) Applications of magnetic nanoparticles in biomedicine J. Phys. D-Appl. Phys.36, R167-R181.
31. Mornet, S., Vasseur, S., Grasset, F., & Duguet, E.(2004) Magnetic nanoparticle design for medical diagnosis and therapy J. Mater. Chem.14, 2161-2175.
32. Lu, Z. H., Prouty, M. D., Guo, Z. H., Golub, V. O., Kumar, C., & Lvov, Y. M.(2005) Magnetic switch of permeability for polyelectrolyte microcapsules embedded with Co@Au nanoparticles Langmuir21, 2042-2050.
33. Hu, S. H., Liu, T. Y., Liu, D. M., & Chen, S. Y.(2007) Controlled pulsatile drug release from a ferrogel by a high-frequency magnetic field Macromolecules40, 6786-6788.
34. Hu, S. H., Chen, S. Y., Liu, D. M., & Hsiao, C. S.(2008) Core/single-crystal-shell nanospheres for controlled drug release via a magnetically triggered rupturing mechanism Adv. Mater.20, 2690-+.
35. Liu, T. Y., Hu, S. H., Liu, T. Y., Liu, D. M., & Chen, S. Y.(2006) Magnetic-sensitive behavior of intelligent ferrogels for controlled release of drug Langmuir22, 5974-5978.
36. Hu, S. H., Liu, T. Y., Liu, D. M., & Chen, S. Y.(2007) Nano-ferrosponges for controlled drug release J. Control. Release121, 181-189.
37. Resendiz-Hernandez, P. J., Rodriguez-Fernandez, O. S., & Garcia-Cerda, L. A.(2008) Synthesis of poly(vinyl alcohol)-magnetite ferrogel obtained by freezing-thawing technique J. Magn. Magn. Mater.320, E373-E376.
38. Chatterjee, J., Haik, Y., & Chen, C. J.(2003) Biodegradable magnetic gel: synthesis and characterization Colloid Polym. Sci.281, 892-896.
39. Qin, J., Asempah, I., Laurent, S., Fornara, A., Muller, R. N., & Muhammed, M.(2009) Injectable Superparamagnetic Ferrogels for Controlled Release of Hydrophobic Drugs Adv. Mater.21, 1354-1357.
40. Thornton, A. J., Alsberg, E., Albertelli, M., & Mooney, D. J.(2004) Shape-defining scaffolds for minimally invasive tissue engineering Transplantation77, 1798-1803.
41. Thornton, A. J., Alsberg, E., Hill, E. E., & Mooney, D. J.(2004) Shape retaining injectable hydrogels for minimally invasive bulking J. Urol.172, 763-768.
42. Park, J., An, K. J., Hwang, Y. S., Park, J. G., Noh, H. J., Kim, J. Y., Park, J. H., Hwang, N. M., & Hyeon, T.(2004) Ultra-large-scale syntheses of monodisperse nanocrystals Nat. Mater.3, 891-895.
43. Qin, J., Laurent, S., Jo, Y. S., Roch, A., Mikhaylova, M., Bhujwalla, Z. M., Muller, R. N., & Muhammed, M.(2007) A high-performance magnetic resonance imaging T-2 contrast agent Adv. Mater. 19, 1874-1878.
44. Drury, J. L. & Mooney, D. J.(2003) Hydrogels for tissue engineering: scaffold design variables and applications Biomaterials24, 4337-4351.
45. Gibson, L. J. & Ashby, M. F. (1997) Cellular solids (Cambridge University Press, Cambridge).
46. Zrinyi, M., Barsi, L., & Buki, A.(1997) Ferrogel: a new magneto-controlled elastic medium Polym. Gels Netw.5, 415-427.
47. Zrinyi, M., Barsi, L., & Buki, A.(1996) Deformation of ferrogels induced by nonuniform magnetic fields J. Chem. Phys.104, 8750-8756.
48. Bouhadir, K. H., Alsberg, E., & Mooney, D. J.(2001) Hydrogels for combination delivery of antineoplastic agents Biomaterials22, 2625-2633.
49. Xiao, Y. & Truskey, G. A.(1996) Effect of receptor-ligand affinity on the strength of endothelial cell adhesion Biophys. J.71, 2869-2884.
50. Rowley, J. A., Madlambayan, G., & Mooney, D. J.(1999) Alginate hydrogels as synthetic extracellular matrix materials Biomaterials20, 45-53.
51. Dainiak, M. B., Kumar, A., Galaev, I. Y., & Mattiasson, B.(2006) Detachment of affinity-captured bioparticles by elastic deformation of a macroporous hydrogel Proc. Natl. Acad. Sci. U. S. A.103, 849-854.
52. Lee, K. Y., Peters, M. C., Anderson, K. W., & Mooney, D. J.(2000) Controlled growth factor release from synthetic extracellular matrices Nature408, 998-1000.
53. Kong, H. J., Hsiong, S., & Mooney, D. J.(2007) Nanoscale cell adhesion ligand presentation regulates nonviral gene delivery and expression Nano Lett.7, 161-166.

All patents and other publications identified in the specification are provided solely for their disclosure prior to the filing date of the present application. Nothing in this regard should be construed as an admission that the inventors are not entitled to antedate such disclosure by virtue of prior invention or for any other reason. All statements as to the date or representation as to the contents of these documents is based on the information available to the applicants and does not constitute any admission as to the correctness of the dates or contents of these documents.

## Claims

1. A composition comprising a matrix material and a magnetic material distributed in the matrix material, wherein the matrix material comprises pores having mean pore diameter in range of about 10 µm to about 1000 µm, and wherein porosity, pore size, pore connectivity and/or specific volume of the matrix material undergoes a change from a first value to a second value in response to an electromagnetic signal.

2. The composition of claim 1, wherein the composition comprises 0.1%-50% of matrix material, 1-90% of magnetic material.

3. The composition of claim 1 or claim 2, wherein the matrix material comprises pores having mean pore diameter in range of about 200 µm to about 1000µm, suitably wherein the matrix material comprises pores having mean pore diameter of 700µm.

4. The composition of any of claims 1-3, wherein the matrix material has porosity of 0.1 to 0.99, suitably wherein the matrix material has porosity of at least 0.9.

5. The composition of any of claims 1-4, wherein the matrix material is a polymer, copolymer, cross-linked polymer or blockpolymer gel.

6. The composition of any of claims 1-5, wherein the magnetic material is a magnetic particle having a size from about 1 nm to 1000 µm, suitably wherein the magnetic material is a magnetic nanoparticle.

7. The composition of any of claims 1-6, wherein the composition comprises a bioactive agent that is optionally covalently linked to the matrix, and suitably wherein the bioactive agent is a therapeutic agent.

8. The composition of any of claims 1-7, wherein the composition comprises a cell, optionally selected from a human cell, a mouse cell or a rat cell.

9. The composition of any of claims 1-8, wherein the matrix material comprises a cell binding molecule, wherein the cell binding molecule is selected from the group consisting of antibodies, intrabodies, antigens, lectins, proteins, peptides, nucleic acids, receptor molecules, ligands for receptors, and any combinations thereof.

10. The composition of claim 9, wherein the cell binding molecule is an integrin-binding peptide and/or wherein the cell binding molecule is a peptide comprising the amino acid sequence Arg-Gly-Asp (RGD).

11. The composition of claim 9 or claim 10, wherein the cell binding molecule is a peptide comprising the amino acid sequence (Gly)₄-Arg-Gly-Asp-Ala-Ser-Ser-Lys-Tyr (SEQ ID NO: 1).

12. The composition of any of claims 1-11, wherein the composition comprises a compound selected from the group consisting of small organic or inorganic molecules; saccharines; oligosaccharides; polysaccharides; peptides; proteins; peptide analogs and derivatives; peptidomimetics; nucleic acids; nucleic acid analogs and derivatives; an extract made from biological materials such as bacteria, plants, fungi, or animal cells; animal tissues; naturally occurring or synthetic compositions; and any combinations thereof.

13. The composition of claim 12, wherein the compound is covalently linked to the matrix, and optionally wherein the compound has biological activity.

14. A method for preparing the composition of any of claims 1-13, method comprising:
(a) preparing a composition comprising a magnetic material; (b) freezing the composition of step (a), wherein freezing temperature is from about -4°C to about - 180°C, suitably from about -10°C to about - 50°C, typically about -20°C; (c) lyophilizing the frozen composition; and (d) swelling the lyophilized composition.

15. A method for controlling the release of a bioactive agent, the method comprising: (a) providing a composition of any of claims 1-13, wherein the composition comprises the bioactive agent; and (b) inducing a change in porosity, pore size, pore connectivity and/or specific volume of the composition via a magnetic field to control the release of the bioactive agent.

## Patentansprüche

1. Zusammensetzung, umfassend ein Matrixmaterial und ein in dem Matrixmaterial verteiltes magnetisches Material, wobei das Matrixmaterial Poren mit einem mittleren Porendurchmesser im Bereich von etwa 10 µm bis etwa 1000 µm umfasst und wobei Porosität, Porengröße, Porenverbindung und/oder spezifisches Volumen des Matrixmaterials als Reaktion auf ein elektromagnetisches Signal eine Veränderung von einem ersten Wert hin zu einem zweiten Wert erfährt.

2. Zusammensetzung nach Anspruch 1, wobei die Zusammensetzung 0,1-50 % Matrixmaterial, 1-90 % magnetisches Material umfasst.

3. Zusammensetzung nach Anspruch 1 oder 2, wobei das Matrixmaterial Poren mit einem mittleren Porendurchmesser im Bereich von etwa 200 µm bis etwa 1000 µm umfasst, geeigneterweise wobei das Matrixmaterial Poren mit einem mittleren Porendurchmesser von 700 µm umfasst.

4. Zusammensetzung nach einem der Ansprüche 1-3, wobei das Matrixmaterial eine Porosität von 0,1 bis 0,99 aufweist, geeigneterweise wobei das Matrixmaterial eine Porosität von wenigstens 0,9 aufweist.

5. Zusammensetzung nach einem der Ansprüche 1-4, wobei das Matrixmaterial ein Polymer-, Copolymer-, vernetztes Polymer- oder Blockpolymergel ist.

6. Zusammensetzung nach einem der Ansprüche 1-5, wobei das magnetische Material ein Magnetpartikel mit einer Größe von etwa 1 nm bis 1000 µm ist, geeigneterweise wobei das magnetische Material ein magnetisches Nanopartikel ist.

7. Zusammensetzung nach einem der Ansprüche 1-6, wobei die Zusammensetzung ein bioaktives Mittel umfasst, das optional kovalent mit der Matrix verbunden ist und geeigneterweise wobei das bioaktive Mittel ein therapeutisches Mittel ist.

8. Zusammensetzung nach einem der Ansprüche 1-7, wobei die Zusammensetzung eine Zelle umfasst, optional ausgewählt aus einer humanen Zelle, einer Mauszelle oder einer Rattenzelle.

9. Zusammensetzung nach einem der Ansprüche 1-8, wobei das Matrixmaterial ein Zellbindungsmolekül umfasst, wobei das Zellbindungsmolekül ausgewählt ist aus der Gruppe bestehend aus Antikörpern, Intrabodys, Antigenen, Lektinen, Proteinen, Peptiden, Nukleinsäuren, Rezeptormolekülen, Liganden für Rezeptoren und beliebigen Kombinationen daraus.

10. Zusammensetzung nach Anspruch 9, wobei das Zellbindungsmolekül ein Integrinbindungspeptid ist und/oder wobei das Zellbindungsmolekül ein Peptid ist, das die Aminosäuresequenz Arg-Gly-Asp (RGD) umfasst.

11. Zusammensetzung nach Anspruch 9 oder 10, wobei das Zellbindungsmolekül ein Peptid ist, das die Aminosäuresequenz (Gly)₄-Arg-Gly-Asp-Ala-Ser-Ser-Lys-Tyr (SEQ ID NO: 1) umfasst.

12. Zusammensetzung nach einem der Ansprüche 1-11, wobei die Zusammensetzung eine Verbindung umfasst, ausgewählt aus der Gruppe bestehend aus kleinen organischen oder anorganischen Molekülen; Saccharinen; Oligosacchariden; Polysacchariden; Peptiden; Proteinen; Peptidanaloga und -derivaten; Peptidomimetika; Nukleinsäuren; Nukleinsäureanaloga und -derivaten; einem Extrakt, hergestellt aus biologischen Materialien, wie etwa Bakterien-, Pflanzen-, Pilz- oder Tierzellen; Tiergewebe; natürlich vorkommenden oder synthetischen Zusammensetzungen; sowie jeglichen Kombinationen daraus.

13. Zusammensetzung nach Anspruch 12, wobei die Verbindung kovalent an die Matrix gebunden ist und optional wobei die Verbindung eine biologische Aktivität aufweist.

14. Verfahren zum Erzeugen der Zusammensetzung nach einem der Ansprüche 1-13, wobei das Verfahren Folgendes umfasst:
(a) Herstellen einer Zusammensetzung, die ein magnetisches Material umfasst; (b) Einfrieren der Zusammensetzung aus Schritt (a), wobei die Einfriertemperatur von etwa - 4 °C bis etwa -180 °C beträgt, geeigneterweise von etwa -10 °C bis etwa -50 °C, typischerweise etwa -20 °C; (c) Lyophilisieren der gefrorenen Zusammensetzung; und (d) Aufquellen der lyophilisierten Zusammensetzung.

15. Verfahren zum Regeln der Freisetzung eines bioaktiven Mittels, wobei das Verfahren Folgendes umfasst: (a) Herstellen einer Zusammensetzung nach einem der Ansprüche 1-13, wobei die Zusammensetzung das bioaktive Mittel umfasst; und (b) Induzieren einer Änderung von Porosität, Porengröße, Porenverbindung und/oder spezifischem Volumen der Zusammensetzung über ein Magnetfeld zum Regeln der Freisetzung des bioaktiven Mittels.

## Revendications

1. Composition comprenant un matériau matriciel et un matériau magnétique réparti dans le matériau matriciel, dans laquelle le matériau matriciel comprend des pores ayant un diamètre moyen des pores compris entre environ 10 µm et environ 1 000 µm, et dans laquelle la porosité, la dimension des pores, la connectivité des pores et/ou le volume massique du matériau matriciel subissent un changement d'une première valeur à une deuxième valeur en réponse à un signal électromagnétique.

2. Composition selon la revendication 1, laquelle composition comprend de 0,1 % à 50 % de matériau matriciel, de 1 % à 90 % de matériau magnétique.

3. Composition selon la revendication 1 ou 2, dans laquelle le matériau matriciel comprend des pores ayant un diamètre moyen des pores compris entre environ 200 µm et environ 1 000 µm, de façon appropriée dans laquelle le matériau matriciel comprend des pores ayant un diamètre moyen des pores de 700 µm.

4. Composition selon l'une quelconque des revendications 1 à 3, dans laquelle le matériau matriciel a une porosité de 0,1 à 0,99, de façon appropriée dans laquelle le matériau matriciel a une porosité d'au moins 0,9.

5. Composition selon l'une quelconque des revendications 1 à 4, dans laquelle le matériau matriciel est un gel de polymère, de copolymère, de polymère réticulé ou de polymère séquencé.

6. Composition selon l'une quelconque des revendications 1 à 5, dans laquelle le matériau magnétique est une particule magnétique ayant une dimension comprise entre environ 1 nm et 1 000 µm, de façon appropriée dans laquelle le matériau magnétique est une nanoparticule magnétique.

7. Composition selon l'une quelconque des revendications 1 à 6, laquelle composition comprend un agent bioactif qui est facultativement lié de façon covalente à la matrice, et de façon appropriée dans laquelle l'agent bioactif est un agent thérapeutique.

8. Composition selon l'une quelconque des revendications 1 à 7, laquelle composition comprend une cellule, facultativement choisie parmi une cellule humaine, une cellule de souris ou une cellule de rat.

9. Composition selon l'une quelconque des revendications 1 à 8, dans laquelle le matériau matriciel comprend une molécule de liaison cellulaire, laquelle molécule de liaison cellulaire est choisie dans le groupe constitué par les anticorps, les intracorps, les antigènes, les lectines, les protéines, les peptides, les acides nucléiques, les molécules réceptrices, les ligands de récepteurs et n'importe quelles combinaisons de ceux-ci.

10. Composition selon la revendication 9, dans laquelle la molécule de liaison cellulaire est un peptide de liaison à l'intégrine et/ou dans laquelle la molécule de liaison cellulaire est un peptide comprenant la séquence d'acides aminés Arg-Gly-Asp (RGD).

11. Composition selon la revendication 9 ou 10, dans laquelle la molécule de liaison cellulaire est un peptide comprenant la séquence d'acides aminés (Gly)₄-Arg-Gly-Asp-Ala-Ser-Ser-Lys-Tyr (SÉQ ID n° : 1).

12. Composition selon l'une quelconque des revendications 1 à 11, laquelle composition comprend un composé choisi dans le groupe constitué par les petites molécules organiques ou inorganiques ; les saccharines ; les oligosaccharides ; les polysaccharides ; les peptides ; les protéines ; les analogues et dérivés peptidiques ; les peptidomimétiques ; les acides nucléiques ; les analogues et dérivés d'acide nucléique ; un extrait produit à partir de matériels biologiques tels que les bactéries, les plantes, les champignons ou les cellules animales ; les tissus animaux ; les compositions naturelles ou synthétiques ; et n'importe quelles combinaisons de ceux-ci.

13. Composition selon la revendication 12, dans laquelle le composé est lié de façon covalente à la matrice, et facultativement dans laquelle le composé a une activité biologique.

14. Procédé de préparation de la composition selon l'une quelconque des revendications 1 à 13, le procédé comprenant :
(a) la préparation d'une composition comprenant un matériau magnétique ; (b) la congélation de la composition de l'étape (a), où la température de congélation est comprise entre environ -4 °C et environ -180 °C, de façon appropriée entre environ -10 °C et environ - 50 °C, généralement d'environ -20 °C ; (c) la lyophilisation de la composition congelée ; et (d) le gonflement de la composition lyophilisée.

15. Procédé de contrôle de la libération d'un agent bioactif, le procédé comprenant : (a) la fourniture d'une composition selon l'une quelconque des revendications 1 à 13, où la composition comprend l'agent bioactif ; et (b) l'induction d'un changement de porosité, de dimension des pores, de connectivité des pores et/ou de volume massique de la composition par le biais d'un champ magnétique pour contrôler la libération de l'agent bioactif.
